(19) Europäisches Patentamt
European Patent Office
Office européen des brevets

(11) EP 1 288 307 A1

(12) **EUROPÄISCHE PATENTANMELDUNG**

(43) Veröffentlichungstag:
**05.03.2003 Patentblatt 2003/10**

(51) Int Cl.[7]: **C12Q 1/527**, C12N 15/52, C12N 9/88

(21) Anmeldenummer: **02018190.5**

(22) Anmeldetag: **20.08.2002**

(84) Benannte Vertragsstaaten:
**AT BE BG CH CY CZ DE DK EE ES FI FR GB GR IE IT LI LU MC NL PT SE SK TR**
Benannte Erstreckungsstaaten:
**AL LT LV MK RO SI**

(30) Priorität: **30.08.2001 DE 10142328**

(71) Anmelder: **Bayer CropScience AG**
**40789 Monheim (DE)**

(72) Erfinder:
- **Koopmann, Edda, Dr.**
  **50670 Köln (DE)**
- **Friedrich, Gabi, Dr.**
  **51377 Leverkusen (DE)**
- **Gutsmann, Volker, Dr.**
  **42799 Leichlingen (DE)**
- **Grimmig, Bernhard, Dr.**
  **40764 Langenfeld (DE)**
- **Kuck, Karl-Heinz, Dr.**
  **40764 Langenfeld (DE)**
- **Vollenbroich, Verena, Dr.**
  **45478 Mühlheim-Ruhr (DE)**

(54) **Verwendung von Fructose-1,6-Bisphospate Aldolase zum Identifizieren von neuen fungizid wirksamen Substanzen**

(57) Die Erfindung betrifft Nukleinsäuren, die für Polypeptide aus Pilzen mit der biologischen Aktivität von Fructose-1,6-Bisphosphate Aldolasen kodieren, die davon kodierten Polypeptide sowie deren Verwendung als Targets für Fungizide und deren Verwendung zum Identifizieren von neuen, füngizid wirksamen Verbindungen sowie Verfahren zum Auffinden von Modulatoren dieser Polypeptide und schließlich transgene Organismen enthaltend für Polypeptide aus Pilzen mit der Funktion einer Fructose-1,6-Bisphosphate Aldolase kodierende Sequenzen.

EP 1 288 307 A1

## Beschreibung

Die Erfindung betrifft Nukleinsäuren, die für Polypeptide aus Pilzen mit der biologischen Aktivität von Fructose-1,6-Bisphosphate Aldolasen kodieren, die davon kodierten Polypeptide sowie deren Verwendung als Targets für Fungizide und deren Verwendung zum Identifizieren von neuen, fungizid wirksamen Verbindungen sowie Verfahren zum Auffinden von Modulatoren dieser Polypeptide und schließlich transgene Organismen enthaltend für Polypeptide aus Pilzen mit der Funktion einer Fructose-1,6-Bisphosphate Aldolase kodierende Sequenzen.

Unerwünschtes Pilzwachstum, dass z.B. in der Landwirtschaft jedes Jahr zu beträchtlichen Schäden führt, kann durch die Verwendung von Fungiziden kontrolliert werden. Die Ansprüche an Fungizide sind dabei hinsichtlich ihrer Wirksamkeit, Kosten und vor allem ihrer Umweltverträglichkeit stetig angestiegen. Es existiert deshalb ein Bedarf an neuen Substanzen bzw. Substanzklassen, die zu leistungsfähigen und umweltverträglichen neuen Fungiziden entwickelt werden können. Im Allgemeinen ist es üblich, in Gewächshaustests nach solchen neuen Leitstrukturen zu suchen. Solche Tests sind jedoch arbeitsintensiv und teuer. Die Anzahl der Substanzen, die im Gewächshaus getestet werden können, ist entsprechend begrenzt. Eine Alternative zu solchen Tests ist die Verwendung von sogenannten Hochdurchsatzverfahren (HTS = high throughput screening). Dabei werden in einem automatisierten Verfahren eine große Anzahl von Einzelsubstanzen hinsichtlich ihrer Wirkung auf Einzelzellen, individuelle Genprodukte oder Gene getestet. Wird für bestimmte Substanzen eine Wirkung nachgewiesen, so können diese in herkömmlichen Screeningverfahren untersucht und gegebenenfalls weiter entwickelt werden.

Vorteilhafte Angriffspunkte für Fungizide werden oft in essentiellen Biosynthesewegen gesucht. Ideale Fungizide sind weiterhin solche Stoffe, die Genprodukte hemmen, die eine entscheidende Bedeutung bei der Ausprägung der Pathogenität eines Pilzes haben. Ein Beispiel für ein solches Fungizid ist z.B: der Wirkstoff Carpropamid, der die Melaninbiosynthese des Pilzes hemmt und so die Ausprägung intakter Appressorien (Haftorgane) verhindert. Es gibt jedoch nur sehr wenige bekannte Genprodukte, die für Pilze eine solche Rolle spielen. Darüber hinaus sind Fungizide bekannt, die durch die Hemmung entsprechender Biosynthesewege zur Auxotrophie der Zielzellen und in der Folge zum Verlust der Pathogenität führen. So führt z.B. die Inhibition der Adenosin Deaminase durch Ethirimol in *Blumeria graminis* zu einer signifikanten Inhibition.

Die Fructose-1,6-Bisphosphate Aldolase (EC 4.1.2.13), auch bekannt als Fructose-1,6-bisphosphat Triosephosphat-Lyase, im Folgenden Aldolase genannt, katalysiert die Aldolspaltung von Fructose-1,6-bisphosphat (1) zu Glycerinaldehyd-3-Phosphat (2) und Dihydroxyacetonphosphat (3).

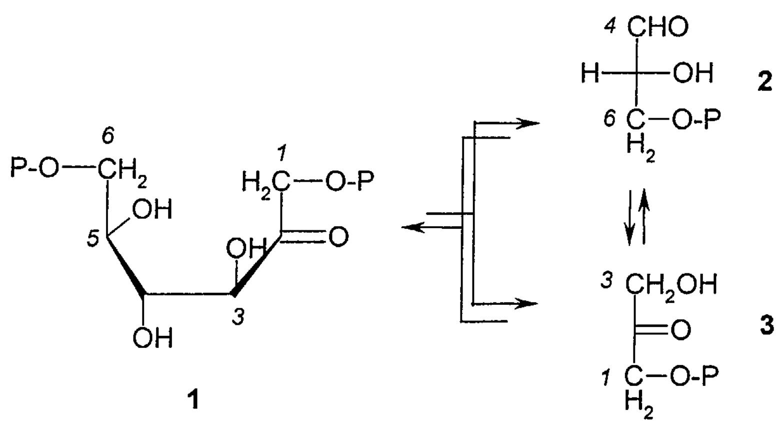

Diese Reaktion ist ein zentraler Schritt in Glykolyse und Gluconeogenese.

Man kann zwei Klassen von Aldolasen aufgrund ihres Reaktionsmechanismus unterscheiden (Marsh und Lebherz, 1992).

Klasse I Aldolasen findet man in Archaebacterien, Eubacterien (z.B. *Micrococcus aerogenes),* höheren Pflanzen, Säugern und Protozoen. Das Enzym besteht aus einem Homotetramer, wobei jede Untereinheit zirka 40 kDa groß ist. Während der Aldolspaltung wird eine Schiffsche Base mit der ε-Aminogruppe eines Lysin-Restes in der aktiven Tasche des Enzyms gebildet.

Klasse II Aldolasen findet man in Archae- und Eubakterien sowie in Pilzen. Sie bestehen als Homodimer aus zwei zirka 40 kDa großen Untereinheiten. Sie besitzen ein charakteristisches $Zn^{2+}$-Ion im aktiven Zentrum.

Gene für die Aldolase wurden aus verschiedenen Organismen kloniert, z.B. aus *E. coli* (Swissprot Accession No.: P71295), *Homo sapiens* (Swissprot Accession No.: P05062), *Arabidopsis thaliana* (Swissprot Accession No.:

P22197) oder *Saccharomyces cerevisiae* (Swissprot Accession No.: P14540).

Die Sequenzähnlichkeit ist innerhalb der Klassen I und II signifikant, während hingegen zwischen Vertretern von Klasse I und II die Sequenzidentität weniger als 20 % beträgt.

Die Aldolase ist aus beiden Klassen ausgezeichnet biochemisch charakterisiert. Kristallstrukturen existieren sowohl von der Klasse II sowie Klasse I Aldolase (Hall et al, 1999; Gamblin et al., 1990).

Trotz dieser hervorragenden Charakterisierung der Fructose-1,6-bisphosphat Aldolase ist das Enzym jedoch niemals als Angriffspunkt für fungizide Wirkstoffe erkannt worden. Die Nutzung dieses interessanten Enzyms beschränkt sich bislang auf die klinische Chemie, in der die Fructose-1,6-bisphosphat Aldolase zur Diagnose von Leberkrankheiten und Myokard-Infarkt benutzt wird (Willnow, 1985).

Im Rahmen der vorliegenden Erfindung wurden die vollständige cDNA Sequenz und die entsprechenden Gene (genomische Sequenz, Chromosom 1, Start 65904-65790 (Exon 1) und 65571-64607 (Exon 2), 65789-65572 (Intron1), siehe SEQ ID NO:1) aus *Ustilago maydis* kodierend für Fructose-1,6-bisphosphat Aldolase isoliert.

Der Brandpilz *Ustilago maydis*, ein Basidiomycet, befällt Maispflanzen. Die Krankheit kommt in allen Maisanbaugebieten vor, erreicht jedoch nur in trockenen Jahren eine größere Bedeutung. Typische Symptome sind die beulenartigen, faustgrossen Anschwellungen (Brandbeulen), die an allen oberirdischen Pflanzenteilen gebildet werden. Die Beulen sind zuerst von einer weiss-grauen, derben Haut überzogen. Beim Aufreissen der Haut wird eine schwarze, zunächst schmierige, später pulvrige Brandsporenmasse frei. Weitere Arten der Gattung Ustilago sind z.B. *U. nuda* (verursacht Gersten- und Weizenflugbrand), *U. nigra* (verursacht Gerstenschwarzbrand), *U. hordei* (verursacht Gerstenhartbrand) und *U. avenae* (verursacht Haferflugbrand).

Im Rahmen der vorliegenden Erfindung wurde mittels Knockout-Analysen im Basidiomyceten *Ustilago maydis* überraschend festgestellt, dass wie im Ascomyceten *Saccharomyces cerevisiae* auch in diesem pflanzenpathogenen Pilz das Enzym essentiell zum Überleben des Organismus ist (siehe auch Schwelberger et al., 1980). Daraus kann geschlossen werden, dass die Fructose-1,6-bisphosphat Aldolase nicht nur für einen spezifischen Pilz, hier *Saccharomyces cerevisiae*, sondern für Pilze im Allgemeinen eine besondere Rolle spielt. Die Fructose-1,6-bisphosphat Aldolase wurde damit erstmals als ein optimales Target für die Suche nach neuen, spezifischen Fungiziden, gerade in pflanzenpathogenen Pilzen, erkannt und damit die Möglichkeit gegeben, mit Hilfe dieses Targets gegebenenfalls völlig neue Leitstrukturen zu identifizieren, die die Fructose-1,6-bisphosphat Aldolase inhibieren und als Fungizide verwendet werden können.

Im Rahmen der vorliegenden Erfindung wurde weiter gefunden, dass die Fructose-1,6-bisphosphat Aldolase zum Identifizieren von Substanzen in geeigneten Testverfahren verwendet werden kann, die die Aktivität des Enzyms beeinflussen. Neben einer Fructose-1,6-bisphosphat Aldolase aus einem phytopathogenen Pilz, die durch ihre Aminosäuresequenz und die dafür kodierende Nukleinsäuresequenz charakterisiert wird, werden auch geeignete Testverfahren zum Identifizieren von Modulatoren des Enzyms zur Verfügung gestellt.

Im Rahmen der vorliegenden Erfindung wurde weiter gefunden, dass die Fructose-1,6-bisphosphat Aldolase auch tatsächlich durch Wirkstoffe inhibiert und ein mit diesen Wirkstoffen behandelter pilzlicher Organismus durch die Behandlung mit diesen Wirkstoffen geschädigt oder abgetötet werden kann, die Inhibitoren der Fructose-1,6-bisphosphat Aldolase also als Fungizide verwendet werden können. In der vorliegenden Erfindung wird beispielsweise gezeigt, dass die Hemmung der Aldolase mit in einem Testsystem identifizierten Substanzen zum Absterben der behandelten Pilze in synthetischen Medien sowie auf der Pflanze führt.

Obwohl bereits bekannt war, dass das für die Fructose-1,6-bisphosphat Aldolase aus *Saccharomyces cerevisiae* kodierende Gen ein essentielles Gen ist, war bislang nicht bekannt, dass die Aldolase in Pilzen ein Zielprotein (ein so genanntes "Target") fungizid wirksamer Substanzen sein kann. So ist ein durch einen Knockout als essentiell erkanntes Gen bzw. Genprodukt nicht als "Target" zu betrachten, wenn keine Möglichkeit besteht, das Genprodukt oder die Expression desselben von außen z.B. durch kleine chemische Moleküle zu beeinflussen. Damit wird in der vorliegenden Erfindung zum ersten Mal gezeigt, dass die Aldolase ein für Pilze, insbesondere für phytopathogene Pilze lebenswichtiges und für Inhibitoren zugängliches Enzym darstellt und deshalb in besonderem Maße dazu geeignet ist, als Zielprotein für die Suche nach weiteren und verbesserten fungizid wirksamen Wirkstoffen verwendet zu werden.

In der vorliegenden Erfindung wird weiterhin zum ersten Mal die Aldolase aus *Ustilago maydis* beschrieben. Die beschriebene Aldolase gehört zur oben beschriebenen Klasse von Aldolasen. Neben der Fructose-1,6-bisphosphat Aldolase aus *Saccharomyces cerevisiae, Neurospora crassa* und *Schizosaccharomyces pombe* sind bislang nur Teilsequenzen anderer Pilze mit einer durchschnittlichen Länge von 400 bis 500 Basenpaaren bekannt geworden und konnten bislang nur potentiell den Fructose-1,6-bisphosphat Aldolasen zugeordnet werden. Die für eine Fructose-1,6-bisphosphat Aldolase kodierende Sequenz aus einem pflanzenpathogenen Basidiomyceten war bislang jedoch noch nicht bekannt, lediglich die Teilsequenz eines humanpathogenen Basidiomyceten war in Datenbanken hinterlegt. Es handelt sich dabei um für Fragmente der Fructose-1,6-bisphosphat Aldolase kodierende DNA aus den pflanzenpathogenen Ascomyceten *Blumeria graminis, Cladosporium fulvum* und *Mycosphaerella graminicola*, die humanpathogenen Pilze *Coccidioides immitis* (ein Ascomycet) und *Cryptococcus neoformans* (ein Basidiomycet) sowie der

nicht pathogenen Pilze *Aspergillus oryzae* (ein Ascomycet) und *Neurospora crassa* (ebenfalls ein Ascomycet) und *E. coli* (siehe Tabelle 1).

| Organismus | Acc. No | Ähnlichkeit | Identität | Länge |
|---|---|---|---|---|
| Aspergillus oryzae | Trembl:Q9HGY9 | 67 | 59 | vollständig |
| **Blumeria graminis 1,3** | **embl: AW787978; AW790281** | **77** | **73** | **teilweise** |
| **Blumeria graminis 2,4** | **embl: AW787979; AW790282** | **72** | **63** | **teilweise** |
| **Cladosporium fulvum** | **EMBL: BE187903** | **63** | **57** | **teilweise** |
| *Coccidioides immitis* | *Trembl: Q9HGT1* | *75* | *68* | *teilweise* |
| *Cryptococcus neoformans* | *embi: AA051839* | *75* | *68* | *teilweise* |
| Kluyvermyces lactis | EMBL: AJ272114 | 72 | 65 | teilweise |
| **Mycosphaerella graminicola** | **embl: AW180694** | **67** | **61** | **teilweise** |
| Neurospora crassa | Swissprot:P53444; (ESTs:EMBL: AI392564; AI398469; AI399236; AI399357; AI399367; BF072733; BF072734;) | 72 | 66 | vollständig |
| Saccharomyces cerevisiae | Swissprot:P14540 | 73 | 65 | vollständig |
| Schizosaccharomyces pombe | Swissprot: P36580 | 73 | 66 | vollständig |
| E. coli | Swissprot:P11604 | 60 | 53 | vollständig |

**Tabelle 1:** Liste der Organismen, deren Sequenzen kodierend für die Fructose-1,6-bisphosphat Aldolase bekannt sind bzw. aus denen Teilsequenzen als kodierend für Teile der Fructose-1,6-bisphosphat Aldolase postuliert wurden. Die Herkunft der Sequenzinformation ist angegeben, ebenso die Ähnlichkeit und Identität der Sequenzen oder Sequenzfragmente zur Fructose-1,6-bisphosphat Aldolase aus *Ustilago maydis* (Aminosäureebene). Pflanzenpathogene Organismen sind fett gezeichnet, humanpathogene Organismen kursiv.

Mittels der bekannten Sequenz aus Saccharomyces sowie der erfindungsgemäßen Sequenz aus *Ustilago maydis* können die putativen, als ESTs bekannten Teilsequenzen als Sequenzen kodierend für Fructose-1,6-bisphosphat Aldolase nun bestätigt werden.

Auf Basis der im Rahmen dieser Erfindung durchgeführten Vergleiche (siehe auch Tabelle 1) lässt sich nunmehr sagen, dass die Aldolasen aus *Ustilago maydis* und aus weiteren Pilzen wie z.B. *Saccharomyces cerevisiae* oder *Schizosaccharomyces pombe* beträchtliche Homologien zueinander aufweisen, weshalb auch zur Fructose-1,6-bisphosphat Aldolase aus *Ustilago maydis* homologe Polypeptide, die von entsprechend homologen Nukleinsäuren kodiert werden, als molekulare Interaktionspartner (Targets) fungizider Wirkstoffe verwendet werden können. Dabei sind vor allem die homologen Nukleinsäuren bzw. Polypeptide aus pflanzenpathogenen Pilzen von Interesse. Besonders bevorzugt können dazu auch pflanzenpathogene Basidiomyceten verwendet werden. Aufgrund der hohen Homologie zwischen den Fructose-1,6-bisphosphat Aldolasen können zum Identifizieren von Inhibitoren des Enzyms auch die Fructose-1,6-bisphosphat Aldolasen aus humanpathogenen Pilzen (siehe Tabelle 1) bzw. die dafür kodierenden Nukleinsäuren verwendet werden. Inhibitoren der Fructose-1,6-bisphosphat Aldolasen können in analoger Weise auch eine Wirkung gegen humanpathogene Pilze entfalten und als Antimycotica verwendet werden.

Die Verwendung von Fructose-1,6-bisphosphat Aldolasen aus Pilzen, besonders aus Ascomyceten, Basidiomyceten und Oomyceten, ganz besonders aus phytopathogenen Pilzen bzw. phytopathogenen Basidiomyceten, und insbesondere aus *Ustilago maydis* zum Identifizieren von fungizid wirksamen Substanzen sind deshalb von der vorliegenden Erfindung voll umfasst.

Besonders bevorzugt handelt es sich bei den genannten homologen Polypeptiden um solche, die zur Aldolase aus *Ustilago maydis* eine Ähnlichkeit von mindestens 60 %, bevorzugt 75 %, besonders bevorzugt 80 %, und besonders bevorzugt von mindestens 95 % über eine Länge von wenigstens 20, vorzugsweise wenigstens 25, besonders bevorzugt wenigstens 30 und ganz besonders bevorzugt wenigstens 100 fortlaufenden Aminosäuren und ganz besonders bevorzugt über die Gesamtlänge aufweisen.

Solche zur Fructose-1,6-bisphosphat Aldolase aus *Ustilago maydis*, insbesondere zum Polypeptid gemäß SEQ ID NO:2 und 3 und SEQ ID NO:5 homologen Polypeptide, die zum Identifizieren von fungiziden Wirkstoffen verwendet werden können, müssen nicht vollständige pilzliche Fructose-1,6-bisphosphat Aldolasen darstellen, sondern können auch nur Fragmente davon sein, solange sie zumindest noch eine biologische Aktivität der vollständigen pilzlichen Fructose-1,6-bisphosphat Aldolasen aufweisen. Polypeptide, die eine gleichartige biologische Aktivität wie eine Aldolase mit einer Aminosäuresequenz gemäß SEQ ID NO:2 und 3 bzw. SEQ ID NO:5 Fructose-1,6-bisphosphat Aldolase ausüben, werden noch als erfindungsgemäß betrachtet. Dabei müssen die erfindungsgemäßen Polypeptide aus den oben genannten Gründen nicht von Fructose-1,6-bisphosphat Aldolasen aus *Ustilago maydis* oder aus phytopathogenen Pilzen ableitbar sein. Als erfindungsgemäß werden vor allem auch solche Polypeptide betrachtet, die Aldolasen beispielsweise der folgenden Pilze entsprechen oder Fragmenten davon, die noch deren biologische Aktivität haben:

Plasmodiophoromycetes, Oomycetes, Chytridiomycetes, Zygomycetes, Ascomycetes, Basidiomycetes und Deuteromycetes, z.B.

Pythium-Arten, wie beispielsweise *Pythium ultimum*, Phytophthora-Arten, wie beispielsweise *Phytophthora infestans*, Pseudoperonospora-Arten, wie beispielsweise *Pseudoperonospora humuli* oder *Pseudoperonospora cubensis*, Plasmopara-Arten, wie beispielsweise *Plasmopara viticola*, Bremia-Arten, wie beispielsweise *Bremia lactucae*, Peronospora-Arten, wie beispielsweise *Peronospora pisi* oder *P. brassicae*, Erysiphe-Arten, wie beispielsweise *Erysiphe graminis*, Sphaerotheca-Arten, wie beispielsweise *Sphaerotheca fuliginea*, Podosphaera-Arten, wie beispielsweise *Podosphaera leucotricha*, Venturia-Arten, wie beispielsweise *Venturia inaequalis*, Pyrenophora-Arten, wie beispielsweise *Pyrenophora teres* oder *P. graminea* (Konidienform: Drechslera, Syn: Helminthosporium), Cochliobolus-Arten, wie beispielsweise *Cochliobolus sativus* (Konidienform: Drechslera, Syn: Helminthosporium), Uromyces-Arten, wie beispielsweise *Uromyces appendiculatus*, Puccinia-Arten, wie beispielsweise *Puccinia recondita*, Sclerotinia-Arten, wie beispielsweise *Sclerotinia sclerotiorum*, Tilletia-Arten, wie beispielsweise *Tilletia caries*; Ustilago-Arten, wie beispielsweise *Ustilago nuda* oder *Ustilago avenae*, Pellicularia-Arten, wie beispielsweise *Pellicularia sasakii*, Pyricularia-Arten, wie beispielsweise *Pyricularia oryzae*, Fusarium-Arten, wie beispielsweise *Fusarium culmorum*, Botrytis-Arten, Septoria-Arten, wie beispielsweise *Septoria nodorum,* Leptosphaeria-Arten, wie beispielsweise *Leptosphaeria nodorum*, Cercospora-Arten, wie beispielsweise *Cercospora canescens*, Alternaria-Arten, wie beispielsweise *Alternaria brassicae* oder Pseudocercosporella-Arten, wie beispielsweise *Pseudocercosporella herpotrichoides.*

Von besonderem Interesse sind z.B. auch *Magnaporthe grisea, Cochliobulus heterostrophus, Nectria hematococcus* and Phytophthora species.

Fungizide Wirkstoffe, die mit Hilfe der erfindungsgemäßen Aldolasen gefunden werden, können auch mit Aldolasen aus humanpathogenen Pilzspezies interagieren, wobei die Interaktion mit den unterschiedlichen in diesen Pilzen vorkommenden Aldolasen nicht immer gleich stark sein muss.

Gegenstand der vorliegenden Erfindung ist deshalb auch die Verwendung von Inhibitoren der Fructose-1,6-bisphosphat Aldolase zum Herstellen von Mitteln zur Behandlung von durch humanpathogene Pilze hervorgerufenen Erkrankungen.

Dabei sind die folgenden humanpathogenen Pilze von besonderem Interesse, die die nachfolgend genannten Krankheitsbilder hervorrufen können: Dermatophyten, wie z.B. Trichophyton spec., Microsporum spec., *Epidermophyton floccosum* oder *Keratomyces ajelloi*, die z.B. Fußmykosen (Tinea pedis) hervorrufen,

Hefen, wie z.B. *Candida albicans*, Soor-Ösophagitis und Dermatitis hervorruft, *Candida glabrata*, *Candida krusei* oder *Cryptococcus neoformans*, die z.B. pulmonale Cryptococcose und auch Torulose hervorrufen können,

Schimmelpilze, wie z.B. *Aspergillus fumigatus, A. flavus*, *A. niger,* die z.B. bronchopulmonale Aspergillose oder Pilzsepsis hervorrufen, Mucor spec., Absidia spec., oder Rhizopus spec., die z.B. Zygomykosen (intravasale Mykosen) hervorrufen, *Rhinosporidium seeberi*, der z.B. chronische granulomatöse Pharyngitis und Tracheitis hervorruft, *Madurella myzetomatis*, der z.B. subkutane Myzetome hervorruft, *Histoplasma capsulatum*, der z.B. retikuloendotheliale Zytomykose und M. Darling hervorruft, *Coccidioides immitis*, der z. B. pulmonale Coccidioidomykose und Sepsis hervorruft, *Paracoccidioides brasiliensis*, der z.B. brasilianische Blastomykose hervorruft, *Blastomyces dermatitidis*, der z.B. Gilchrist-Krankheit und nordamerikanische Blastomykose hervorruft, *Loboa loboi*, der z.B. Keloid-Blastomykose und Lobo's Krankheit hervorruft, und *Sporothrix schenckii*, der z.B. Sporotrichose (granulomatöse Hautmykose) hervorruft.

Fungizide Wirkstoffe, die mit Hilfe der erfindungsgemäßen Aldolasen gefunden werden, können also auch mit Aldolasen aus anderen zahlreichen phytopathogenen Pilzspezies interagieren, wobei die Interaktion mit den unterschiedlichen in diesen Pilzen vorkommenden Aldolasen nicht immer gleich stark sein muss. Dies erklärt unter anderem die beobachtete Selektivität der an diesem Enzym wirksamen Substanzen.

Gegenstand der vorliegenden Erfindung sind deshalb Nukleinsäuren, die für vollständige Fructose-1,6-bisphosphat Aldolasen aus pflanzenpathogenen Pilzen kodieren mit Ausnahme der in Tabelle 1 aufgeführten Sequenzfragmente aus *Blumeria graminis, Cladosporium fulvum* und *Mycosphaerella graminicola* mit den unter den angegebenen Accession Numbers hinterlegten Sequenzen.

Gegenstand der vorliegenden Erfindung sind insbesondere Nukleinsäuren, die für Fructose-1,6-bisphosphat Aldolasen aus Basidiomyceten kodieren, bevorzugt aus pflanzenpathogenen Basidiomyceten und ganz besonders bevorzugt aus der Gattung Ustilago.

Gegenstand der vorliegenden Erfindung sind ganz besonders bevorzugt Nukleinsäuren, die für Fructose-1,6-bisphosphat Aldolase aus *Ustilago maydis* kodieren.

Gegenstand der vorliegenden Erfindung sind insbesondere bevorzugt Nukleinsäuren aus *Ustilago maydis* gemäß SEQ ID NO:1 und 4, die für ein Polypeptid gemäß SEQ ID NO: 2 und 3 bzw. SEQ ID NO:5 oder aktive Fragmente davon kodieren.

Von besonderem Interesse ist dabei das Aminosäurefragment umfassend die Aminosäuren 80 bis 220, das wiederum die für die Fructose-1,6-bisphosphat Aldolase bedeutsame Region 107-110 umfasst, welches wichtig für die Zink-Bindung ist und damit zum aktiven Zentrum gehört. Zwei so genannte Prosite Motive (Hofmann et al., 1999) finden sich ebenfalls in diesem Bereich, wobei sich Motiv (I) über die Aminosäuren 99-111 erstreckt, Motiv (II) über die Aminosäuren 171-182. Ein so genanntes Prosite Motiv wird bei einer Suche gestützt auf Protein Sequenzen und funktionelle Domänen mit dem Programm PROSITE identifiziert und ist geeignet, die Funktion eines Genprodukts vorherzusagen. Ein Prosite Motiv ist damit typisch für eine bestimmte Enzymklasse. Ein Prosite Motiv stellt letztlich die Komponenten einer Konsensussequenz dar, sowie Abstände zwischen den beteiligten Aminosäuren.

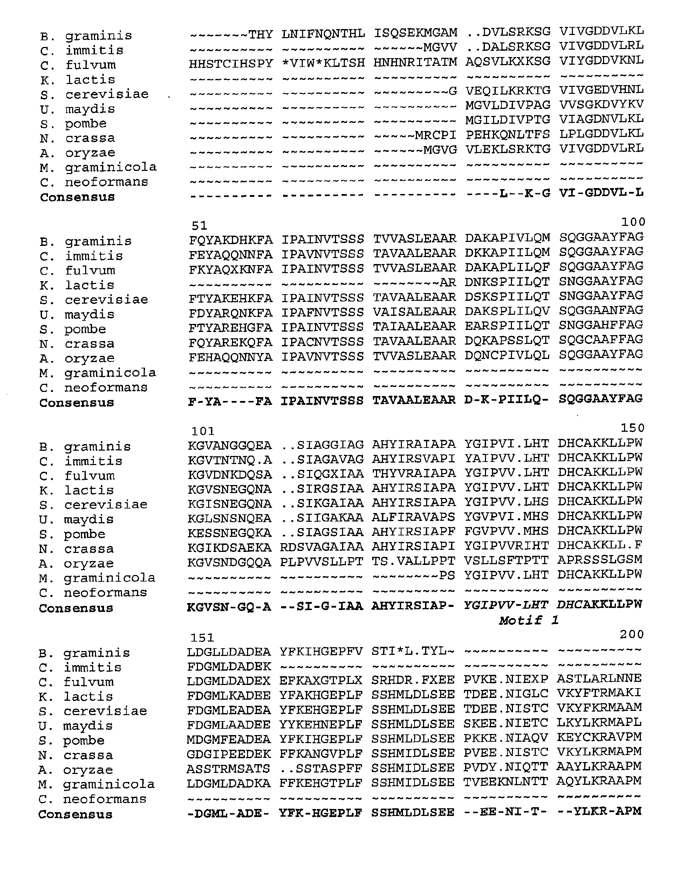

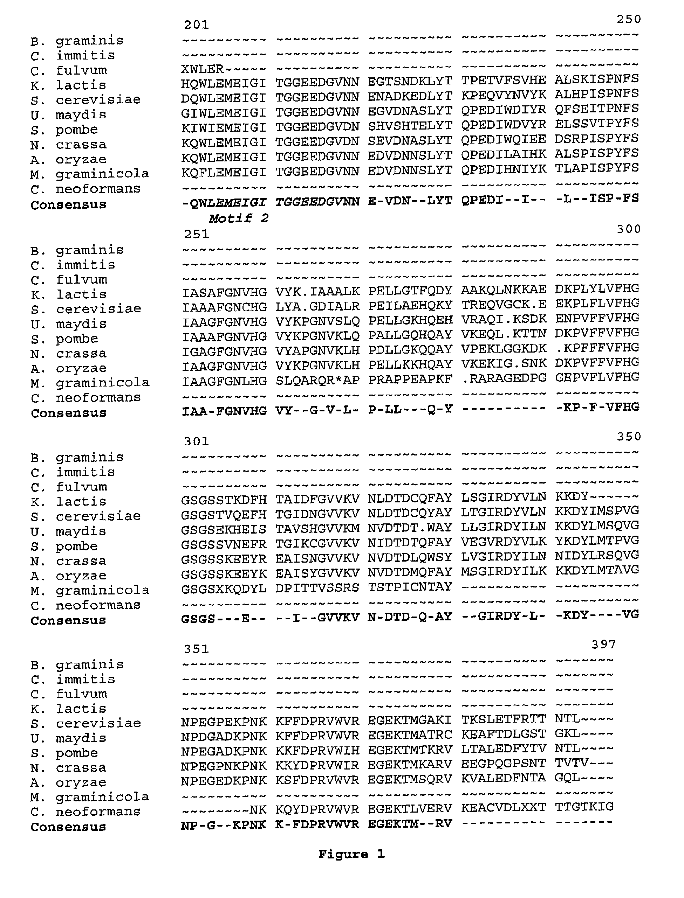

Die mit Hilfe der nun vorliegenden Sequenzdaten in einer entsprechenden Suche identifizierten Motive können wie folgt definiert werden (vgl. auch Abbildung 1):

**Prosite Motiv (I): [FYVMT]-x(1,3)-[LIVMH]-[APNT]-[LIVM]-x(1,2) [LIVM]-H-x-D-H-[GACH]**

H-x-D-H ist dabei für die Bindung des katalytischen Zn-Ions geeignet. Ein Vergleich mit der Abbildung 1 zeigt, dass sich das Konsensusmotiv (II) im vorliegenden Fall auch spezifisch mit

**-(YGIPVV)-x-(LHTDHC)**

beschreiben lässt, wobei x für eine Position steht, an der jede Aminosäure akzeptiert wird oder auch keine Aminosäure steht.

**Prosite Motiv (II): [LIVM]-E-x-E-[LIVM]-G-x(2)-[GM]-[GSTA]-x-E**

Ein Vergleich mit der Abbildung 1 zeigt, dass sich das Konsensusmotiv (II) im vorliegenden Fall auch spezifisch mit

**-(LEMEIGITGGEEDGV)-**

beschreiben lässt.

Die oben genannten Prosite Motive bzw. die spezifischen Konsensusmotive sind typisch für die erfindungsgemäßen Polypeptide, die durch diese Konsensussequenzen strukturell definiert werden können und dadurch identifizierbar sind.

Demgemäß sind auch vor allem solche Nukleinsäuren Gegenstand der vorliegenden Erfindung, die für Polypeptide kodieren, die bevorzugt sowohl das Prosite Motiv (I) als auch das Prosite Motiv (II) umfassen. Besonders bevorzugt umfassen die von den erfindungsgemäßen Nukleinsäuren kodierten Polypeptide sowohl das Konsensusmotiv (I) als auch das Konsensusmotiv (II).

Bei den erfindungsgemäßen Nukleinsäuren handelt es sich insbesondere um einzelsträngige oder doppelsträngige Desoxyribonukleinsäuren (DNA) oder Ribonukleinsäuren (RNA). Bevorzugte Ausführungsformen sind Fragmente genomischer DNA, die Introns enthalten können, und cDNAs.

Bevorzugt handelt es sich bei den erfindungsgemäßen Nukleinsäuren um DNA-Fragmente, die der cDNA der erfindungsgemäßen Nukleinsäuren entsprechen.

Besonders bevorzugt umfassen die erfindungsgemäßen Nukleinsäuren eine Sequenz aus Pilzen ausgewählt aus

a) der Sequenz gemäß SEQ ID NO: 1 und SEQ ID NO: 4

b) Sequenzen, die für ein Polypeptid kodieren, welches die Aminosäuresequenz gemäß SEQ ID NO: 2 und 3 sowie SEQ ID NO: 5 umfasst,

c) Sequenzen, die für ein Polypeptid kodieren, welches die Konsensusmotive - (YGIPVV)-x-(LHTDHC)- und -(LEMEIGITGGEEDGV)- umfasst,

d) zumindest 14 Basenpaare langen Teilsequenzen der unter a) bis c) definierten Sequenzen,

e) Sequenzen, welche an die unter a) bis c) definierten Sequenzen bei einer Hybridisierungstemperatur von 35-52°C hybridisieren,

f) Sequenzen, welche eine zumindest eine 60 %ige, bevorzugt eine 80 %ige, besonders bevorzugt eine 90 %ige und ganz besonders bevorzugt eine 95 % ige Identität mit den unter a) bis c) definierten Sequenzen aufweisen,

g) Sequenzen, welche zu den unter a) bis c) definierten Sequenzen komplementär sind, und

h) Sequenzen, welche aufgrund der Degeneriertheit des genetischen Codes für dieselbe Aminosäuresequenz codieren wie die unter a) bis c) definierten Sequenzen.

Eine ganz besonders bevorzugte Ausführungsform der erfindungsgemäßen Nukleinsäuren stellt ein cDNA-Molekül mit der Sequenz gemäß SEQ ID NO:1 oder SEQ ID NO:4 kodierend für Fructose-1,6-bisphosphat Aldolase mit der SEQ ID NO:2 und 3 oder SEQ ID NO: 5 aus *Ustilago maydis* dar.

Der Begriff "Identität", wie er hierin verwendet wird, bezieht sich auf die Zahl von Sequenzpositionen die in einem Alignment identisch sind. Sie wird meist in Prozent der Alignment Länge angegeben.

Der Begriff "Ähnlichkeit", wie er hierin verwendet wird, setzt dagegen die Definition einer Ähnlichkeitsmetrik voraus, also eines Maßes dafür, als wie ähnlich man beispielsweise ein Valin zu einem Threonin oder zu einem Leucin annehmen möchte.

Der Begriff "Homologie", wie er hierin verwendet wird, bedeutet wiederum evolutionäre Verwandtschaft. Zwei homologe Proteine haben sich aus einer gemeinsamen Vorläufersequenz entwickelt. Der Begriff hat nicht unbedingt etwas mit Identität oder Ähnlichkeit zu tun, abgesehen davon, dass homologe Sequenzen meist ähnlicher sind (oder in einem Alignment mehr identische Positionen besitzen) als nicht-homologe Sequenzen.

Der Ausdruck "vollständige" Fructose-1,6-bisphosphat Aldolase, wie er hierin verwendet wird, beschreibt die Fructose-1,6-bisphosphat Aldolasen, die von der vollständigen kodierenden Region einer Transkriptionseinheit kodiert wird, beginnend mit dem ATG-Startcodon und umfassend alle informationstragenden Exonbereiche des im Herkunftsorganismus vorliegenden, für Fructose-1,6-bisphosphat Aldolase kodierenden Gens, sowie die für eine korrekte Termination der Transkription nötigen Signale.

Der Ausdruck "aktives Fragment" wie er hierin verwendet wird, beschreibt nicht mehr vollständige Nukleinsäuren kodierend für Fructose-1,6-bisphosphat Aldolase, die aber noch für Enzyme mit der biologischen Aktivität einer Fructose-1,6-bisphosphat Aldolase kodieren, und die eine für eine Fructose-1,6-bisphosphat Aldolase charakteristische Reaktion wie vorne beschrieben katalysieren können. Solche Fragmente sind kürzer als die oben beschriebenen vollständigen, für die Fructose-1,6-bisphosphat Aldolase kodierenden Nukleinsäuren. Dabei können sowohl an den 3'und/oder 5'-Enden der Sequenz Nukleinsäuren entfernt worden sein, es können aber auch Teile der Sequenz deletiert, d.h. entfernt worden sein, die die biologische Aktivität der Fructose-1,6-bisphosphat Aldolase nicht entscheidend beeinträchtigen. Eine geringere oder gegebenenfalls auch eine erhöhte Aktivität, die aber noch die Charakterisierung bzw. Verwendung des resultierenden Fructose-1,6-bisphosphat Aldolase Fragments gestattet, wird dabei als ausreichend im Sinne des hier verwendeten Ausdrucks verstanden. Der Ausdruck "aktives Fragment" kann sich ebenso auf die Aminosäuresequenz der Fructose-1,6-bisphosphat Aldolase beziehen und gilt dann analog den obigen Ausführungen für solche Polypeptide, die im Vergleich zur oben definierten vollständigen Sequenz bestimmte Teile nicht mehr enthalten, wobei die biologische Aktivität des Enzyms jedoch nicht entscheidend beeinträchtigt ist. Die bevorzugte Länge dieser Fragmente beträgt 420 Nukleobasen, bevorzugt 660 Nukleobasen, ganz besonders bevorzugt 1056 Nukleobasen, bzw. 140 Aminosäuren, bevorzugt 220 Aminosäuren, ganz besonders bevorzugt 352 Aminosäuren.

Der Ausdruck "Gen", wie er hierin verwendet wird, ist die Bezeichnung für einen Abschnitt aus dem Genom einer Zelle, der für die Synthese einer Polypeptid-Kette verantwortlich ist.

Der Ausdruck "hybridisieren", wie er hierin verwendet wird, beschreibt den Vorgang, bei welchem ein einzelsträngiges Nukleinsäuremolekül mit einem komplementären Strang eine Basenpaarung eingeht. Auf diese Weise können ausgehend von der hierin genannten oder ableitbaren Sequenzinformation beispielsweise DNA-Fragmente aus anderen phytopathogenen Pilzen als *Ustilago maydis* isoliert werden, welche für Aldolasen kodieren, welche dieselben oder ähnliche Eigenschaften einer der erfindungsgemäßen Aldolasen aufweisen.

Hybridisierungsbedingungen werden nach folgender Formel näherungsweise berechnet:

Die Schmelztemperatur

$$Tm = 81{,}5°C + 16{,}6\ \{\log[c(Na^+)]\} + 0{,}41(\%\ G + C) - (500/n)$$

(Lottspeich & Zorbas, 1998).

Dabei ist c die Konzentration und n die Länge des hybridisierenden Sequenzabschnitts in Basenpaaren. Für eine Sequenz >100 bp entfällt der Ausdruck 500/n. Mit höchster Stringenz wird bei einer Temperatur 5-15°C unterhalb Tm und einer Ionenstärke von 15 mM $Na^+$ (entspricht 0.1 x SSC) gewaschen. Wird eine RNA-Probe zur Hybridisierung verwendet, so ist der Schmelzpunkt um 10-15°C höher.

Bevorzugte Hybridisierungsbedingungen sind nachstehend angegeben:

Hybridisierungslösung: DIG Easy Hyb (Roche, ZZ) Hybridisierungstemperatur: 37°C bis 50°C, bevorzugt 42°C (DNA-DNA), 50°C (DNA-RNA).

1. Waschschritt: 2 x SSC, 0,1 % SDS 2 x5 min bei Raumtemperatur;
2. Waschschritt: 1 x SSC, 0,1 % SDS 2 x 15 min bei 50°C; bevorzugt 0,5 x SSC, 0,1 % SDS 2 x 15 min bei 65°C; besonders bevorzugt 0,2 x SSC, 2 x 15min bei 68°C.

Der Grad der Identität der Nukleinsäuren wird vorzugsweise bestimmt mit Hilfe des Programms NCBI BLASTN Version 2.0.4. (Altschul et al. 1997).

Der Ausdruck "heterologer Promotor", wie er hierin verwendet wird, bezieht sich auf einen Promotor, der andere Eigenschaften als derjenige Promotor aufweist, der im Ursprungsorganismus die Expression des betreffenden Gens kontrolliert.

Die Auswahl von heterologen Promotoren ist davon abhängig, ob zur Expression pro- oder eukaryotische Zellen oder zellfreie Systeme verwendet werden. Beispiele für heterologe Promotoren sind der 35S Promoter des Blumenkohlmosaikvirus für pflanzliche Zellen, der Promoter der Alkoholdehydrogenase für Hefezellen, die T3-, T7- oder SP6-Promotoren für prokaryotische Zellen oder zellfreie Systeme, sowie gewebespezifische Promotoren aus phytopathogenen Pilzen, z.B. der spezifische Promotor der erfindungsgemäß zu verwendenden Aldolase.

Gegenstand der vorliegenden Erfindung sind ferner Vektoren, die eine erfindungsgemäße Nukleinsäure, eine erfindungsgemäße regulatorische Region oder ein erfindungsgemäßes DNA-Konstrukt enthalten.

Als Vektoren können alle in molekularbiologischen Laboratorien verwendete Phagen, Plasmide, Phagmide, Phasmide, Cosmide, YACs, BACs, künstliche Chromosomen oder Partikel, die für einen Partikelbeschuss geeignet sind, verwendet werden.

Ein bevorzugter Vektor ist pET15b (Novagen).

Gegenstand der vorliegenden Erfindung sind auch Wirtszellen, die eine erfindungsgemäße Nukleinsäure, ein erfindungsgemäßes DNA-Konstrukt oder einen erfindungsgemäßen Vektor enthalten.

Der Ausdruck "Wirtszelle", wie er hierin verwendet wird, bezieht sich auf Zellen, die natürlicherweise die erfindungsgemäßen Nukleinsäuren nicht enthalten.

Als Wirtszellen eignen sich sowohl prokaryotische Zellen, vorzugsweise *E. coli,* als auch eukaryotische Zellen, wie Zellen von *Saccharomyces cerevisiae*, *Pichia pastoris*, phytopathogenen Pilzen, Pflanzen, Froschoozyten und Zelllinien von Säugern und Insekten.

Gegenstand der vorliegenden Erfindung sind weiterhin Polypeptide mit der biologischen Aktivität von Fructose-1,6-bisphosphat Aldolasen, die von den erfindungsgemäßen Nukleinsäuren kodiert werden.

Bevorzugt umfassen die erfindungsgemäßen Polypeptide eine Aminosäuresequenz ausgewählt aus

a) der Sequenz gemäß SEQ ID NO:2 und 3 und SEQ ID NO:5,

b) Sequenzen umfassend die Konsensusmotive -(YGIPVV)-x-(LHTDHC)- und -(LEMEIGITGGEEDGV)-,

c) zumindest 15 Aminosäuren lange Teilsequenzen der unter a) und b) definierten Sequenzen,

d) Sequenzen, welche eine zumindest 60 % ige, bevorzugt eine 80 % ige und besonders bevorzugt eine 90 % ige Identität mit den unter a) und b) definierten Sequenzen haben, und

e) Sequenzen, welche die gleiche biologische Aktivität aufweisen wie die unter a) und b) definierten Sequenzen.

Der Ausdruck "Polypeptide", wie er hierin verwendet wird, bezieht sich sowohl auf kurze Aminosäureketten,

die gewöhnlich als Peptide, Oligopeptide oder Oligomere bezeichnet werden, als auch auf längere Aminosäureketten, die gewöhnlich als Proteine bezeichnet werden. Er umfasst Aminosäureketten, die entweder durch natürliche Prozesse, wie posttranslationale Prozessierung, oder durch chemische Verfahren, die Stand der Technik sind, modifiziert sein können. Solche Modifikationen können an verschiedenen Stellen und mehrfach in einem Polypeptid vorkommen, wie beispielsweise am Peptid-Rückgrat, an der Aminosäure-Seitenkette, am Aminound/oder am Carboxy-Terminus. Sie umfassen beispielsweise Acetylierungen, Acylierungen, ADP-Ribosylierungen, Amidierungen, kovalente Verknüpfungen mit Flavinen, Häm-Anteilen, Nukleotiden oder Nukleotid-Derivaten, Lipiden oder Lipid-Derivaten oder Phosphatidylinositol, Cyclisierungen, Disulfidbrückenbildungen, Demethylierungen, Cystin-Bildungen, Formylierungen, gamma-Carboxylierungen, Glycosylierungen, Hydroxylierungen, Iodierungen, Methylierungen, Myristoylierungen, Oxidationen, proteolytische Prozessierungen, Phosphorylierungen, Selenoylierungen und tRNA-vermittelte Additionen von Aminosäuren.

Die erfindungsgemäßen Polypeptide können in der Form "reifer" Proteine oder als Teile größerer Proteine, z.B. als Fusionsproteine, vorliegen. Weiterhin können sie Sezernierungs- oder "Leader"-Sequenzen, Pro-Sequenzen, Sequenzen, die eine einfache Reinigung ermöglichen, wie mehrfache Histidin-Reste, oder zusätzliche stabilisierende Aminosäuren aufweisen. Die erfindungsgemäßen Proteine können ebenfalls so vorliegen, wie sie natürlicherweise in ihrem Herkunftsorganismus vorliegen, aus dem sie zum Beispiel direkt gewonnen werden können.

Der Begriff "vollständige Aldolase" wie er hierin verwendet wird, beschreibt eine Aldolase, die kodiert wird von einer vollständigen kodierenden Region einer Transkriptionseinheit beginnend mit dem ATG-Startcodon und umfassend alle informationstragenden Exonbereiche des im Herkunftsorganismus vorliegenden, für die Aldolase kodierenden Gens, sowie für eine korrekte Termination der Transkription nötigen Signale.

Die erfindungsgemäßen Polypeptide können im Vergleich zu den entsprechenden Regionen von natürlich vorkommenden Aldolasen Deletionen oder Aminosäuresubstitutionen aufweisen, solange sie zumindest noch eine biologische Aktivität der vollständigen Aldolasen ausüben. Konservative Substitutionen sind bevorzugt. Solche konservativen Substitutionen umfassen Variationen, wobei eine Aminosäure durch eine andere Aminosäure aus der folgenden Gruppe ersetzt wird:

1. Kleine aliphatische, nicht-polare oder wenig polare Reste: Ala, Ser, Thr, Pro und Gly;
2. Polare, negativ geladene Reste und deren Amide: Asp, Asn, Glu und Gln;
3. Polare, positiv geladene Reste: His, Arg und Lys;
4. Große aliphatische, nicht-polare Reste: Met, Leu, Ile, Val und Cys; und
5. Aromatische Reste: Phe, Tyr und Trp.

Die folgende Liste zeigt bevorzugte konservative Substitutionen:

| Ursprünglicher Rest | Substitution |
|---|---|
| Ala | Gly, Ser |
| Arg | Lys |
| Asn | Gln, His |
| Asp | Glu |
| Cys | Ser |
| Gln | Asn |
| Glu | Asp |
| Gly | Ala, Pro |
| His | Asn, Gln |
| Ile | Leu, Val |
| Leu | Ile, Val |
| Lys | Arg, Gln, Glu |
| Met | Leu, Tyr, Ile |
| Phe | Met, Leu, Tyr |
| Ser | Thr |

(fortgesetzt)

| Ursprünglicher Rest | Substitution |
|---|---|
| Thr | Ser |
| Trp | Tyr |
| Tyr | Trp, Phe |
| Val | Ile, Leu |

Gegenstand der vorliegenden Erfindung ist somit auch die Verwendung von Polypeptiden, welche zumindest eine biologische Aktivität einer Fructose-1,6-bisphosphat Aldolase ausüben und eine Aminosäuresequenz umfassen, die eine zumindest 60 %ige, bevorzugt eine 80 %ige Identität und besonders bevorzugt eine 95% ige Identität mit der Sequenz aus *Ustilago maydis* gemäß SEQ ID NO: 1 aufweisen.

Der Ausdruck "biologische Aktivität einer Aldolase", wie er hierin verwendet wird, bedeutet die Fähigkeit, die Aldolspaltung von Fructose-1,6-bisphosphat zu katalysieren.

Die erfindungsgemäßen Nukleinsäuren können auf die übliche Weise hergestellt werden. Beispielsweise können die Nukleinsäuremoleküle vollständig chemisch synthetisiert werden. Man kann auch kurze Stücke der erfindungsgemäßen Nukleinsäuren chemisch synthetisieren und solche Oligonukleotide radioaktiv oder mit einem Fluoreszenzfarbstoff markieren. Die markierten Oligonukleotide können auch verwendet werden, um von mRNA z.B. aus phytopathogenen Pilzen hergestellte cDNA-Banken zu durchsuchen. Klone, an die die markierten Oligonukleotide hybridisieren, werden zur Isolierung der betreffenden DNA-Fragmente ausgewählt. Nach der Charakterisierung der isolierten DNA erhält man auf einfache Weise die erfindungsgemäßen Nukleinsäuren.

Die erfindungsgemäßen Nukleinsäuren können auch mittels PCR-Verfahren unter Verwendung chemisch synthetisierter Oligonukleotide hergestellt werden.

Der Ausdruck "Oligonukleotid(e)", wie er hierin verwendet wird, bedeutet DNA-Moleküle, die aus 10 bis 50 Nukleotiden, vorzugsweise 15 bis 30 Nukleotiden, bestehen. Sie werden chemisch synthetisiert und können als Sonden verwendet werden.

Zur Herstellung der erfindungsgemäßen Polypeptide, insbesondere des von der Nukleinsäuresequenz gemäß SEQ ID NO: 1 kodierten Polypeptids, können außerdem Wirtszellen, die erfindungsgemäßen Nukleinsäuren enthalten, unter geeigneten Bedingungen kultiviert werden. Die gewünschten Polypeptide können danach auf übliche Weise aus den Zellen oder dem Kulturmedium isoliert werden. Die Polypeptide können auch in *in vitro*-Systemen hergestellt werden.

Zur Herstellung der erfindungsgemäßen Aldolase aus *Ustilago maydis* kann das Gen z.B. rekombinant in *Escherichia coli* exprimiert und aus *E. coli* Zellen eine Enzympräparation hergestellt werden.

Ein mögliches Reinigungsverfahren der Aldolase basiert auf präparativer Elektrophorese, FPLC, HPLC (z.B. unter Anwendung von Gelfiltrations-, Reversphasenoder leicht hydrophoben Säulen), Gelfiltration, differentieller Präzipitation, Ionenaustausch-Chromatographie oder Affinitätschromatographie.

Ein schnelles Verfahren zum Isolieren der erfindungsgemäßen Polypeptide, die von Wirtszellen unter Verwendung einer erfindungsgemäß zu verwendenden Nukleinsäure synthetisiert werden, beginnt mit der Expression eines Fusionsproteins, wobei der Fusionspartner auf einfache Weise affinitätsgereinigt werden kann. Der Fusionspartner kann beispielsweise Sechs-His-Tag sein. Das Fusionsprotein kann dann an einer Nickel-NTA-Affinitätssäule gereinigt werden. Der Fusionspartner kann durch partielle proteolytische Spaltung beispielsweise an Linkern zwischen dem Fusionspartner und dem zu reinigenden erfindungsgemäßen Polypeptid abgetrennt werden. Der Linker kann so gestaltet werden, dass er Ziel-Aminosäuren, wie Arginin- und Lysin-Reste einschließt, die Stellen für eine Spaltung durch Trypsin definieren. Um solche Linker zu erzeugen, können Standard-Klonierungsverfahren unter Verwendung von Oligonukleotiden angewendet werden.

Weitere mögliche Reinigungsverfahren basieren wiederum auf präparativer Elektrophorese, FPLC, HPLC (z. B. unter Anwendung von Gelfiltrations-, Reversphasenoder leicht hydrophoben Säulen), Gelfiltration, differentieller Präzipitation, Ionenaustausch-Chromatographie und Affinitätschromatographie.

Die Ausdrücke "Isolierung oder Reinigung", wie sie hierin verwendet werden, bedeuten, dass die erfindungsgemäßen Polypeptide von anderen Proteinen oder anderen Makromolekülen der Zelle oder des Gewebes abgetrennt werden. Vorzugsweise ist eine die erfindungsgemäßen Polypeptide enthaltende Zusammensetzung hinsichtlich des Proteingehalts gegenüber einer Präparation aus den Wirtszellen mindestens 10-fach und besonders bevorzugt mindestens 100-fach angereichert.

Die erfindungsgemäßen Polypeptide können auch ohne Fusionspartner mit Hilfe von Antikörpern, die an die Polypeptide binden, affinitätsgereinigt werden.

Gegenstand der vorliegenden Erfindung sind insbesondere auch Verfahren zum Auffinden von chemischen

Verbindungen, die mit der Aldolase interagieren und deren Eigenschaften verändern. Aufgrund der wichtigen Funktion der Aldolase stellen Modulatoren, die die Aktivität beeinflussen, neue fungizide Wirkstoffe dar. Modulatoren können Agonisten oder Antagonisten bzw. Inhibitoren oder Aktivatoren sein.

Gegenstand der vorliegenden Erfindung ist ebenfalls die Verwendung der erfindungsgemäßen Polypeptide in Verfahren zum Auffinden von chemischen Verbindungen, die an die Fructose-1,6-bisphosphat Aldolase binden und deren Eigenschaften verändern.

Auf Grund der Eigenschaft, als Inhibitoren der Aldolase von Pilzen, insbesondere phytopathogener Pilze zu wirken, können mittels geeigneter Verfahren gefundene Verbindungen, die die Fructose-1,6-bisphosphat Aldolase inhibieren, auch als gegebenenfalls markierte Kompetitoren in Verfahren zum Auffinden von weiteren Inhibitoren der Aldolase aus Pilzen verwendet werden, die nicht dieser Gruppe von Verbindungen angehören müssen.

Die Verwendung der erfindungsgemäßen Nukleinsäuren bzw. Polypeptide in einem erfindungsgemäßen Verfahren ermöglicht das Auffinden von Verbindungen, die an die erfindungsgemäßen Polypeptide binden. Diese können dann als Fungizide, z.B. bei Pflanzen oder als antimycotische Wirkstoffe bei Menschen und Tieren angewandt werden. Beispielsweise werden Wirtszellen, die die erfindungsgemäßen Nukleinsäuren enthalten und die entsprechenden Polypeptide exprimieren oder die Genprodukte selbst mit einer Verbindung oder einem Gemisch von Verbindungen unter Bedingungen in Kontakt gebracht, die die Wechselwirkung zumindest einer Verbindung mit den Wirtszellen, den Rezeptoren oder den einzelnen Polypeptiden erlauben.

Insbesondere ist ein Verfahren Gegenstand der vorliegenden Erfindung, dass zur Identifizierung von fungiziden Wirkstoffen geeignet ist, die mit Polypeptide aus Pilzen mit der biologischen Aktivität einer Fructose-1,6-bisphosphat Aldolase interagieren, bevorzugt mit Fructose-1,6-bisphosphat Aldolase aus phytopathogenen Pilzen, besonders bevorzugt mit Fructose-1,6-bisphosphat Aldolase aus Ustilago und dazu homologe Polypeptide, die die vorstehend genannte Konsensussequenz aufweisen. Die Verfahren können jedoch auch mit einem zur Fructose-1,6-bisphosphat Aldolase homologen Polypeptid aus einer anderen als den hier genannten Spezies durchgeführt werden. Verfahren, die andere als die erfindungsgemäße Fructose-1,6-bisphosphat Aldolase verwenden, sind von der vorliegenden Erfindung umfasst.

Viele Testsysteme, die die Prüfung von Verbindungen und natürlichen Extrakten zum Ziel haben, sind auf hohe Durchsatzzahlen ausgerichtet, um die Zahl der untersuchten Substanzen in einem gegebenen Zeitraum zu maximieren. Testsysteme, die auf zellfreiem Arbeiten beruhen, brauchen gereinigtes oder semi-gereinigtes Protein. Sie sind geeignet für eine "erste" Prüfung, die in erster Linie darauf abzielt, einen möglichen Einfluss einer Substanz auf das Zielprotein zu detektieren.

Um Modulatoren aufzufinden, kann ein synthetischer Reaktionsmix (z.B. Produkte der in vitro Transkription) oder ein zellulärer Bestandteil, wie eine Membran, ein Kompartiment oder irgendeine andere Präparation, die die erfindungsgemäßen Polypeptide enthält, zusammen mit einem markierten Substrat oder Liganden der Polypeptide in Gegenwart und Abwesenheit einer Kandidatenverbindung bzw. eines Kandidatenmoleküls, das ein Agonist oder Antagonist sein kann, inkubiert werden. Die Fähigkeit des Kandidatenmoleküls die Aktivität der erfindungsgemäßen Polypeptide zu erhöhen oder zu hemmen, wird erkennbar an einer erhöhten oder verringerten Bindung des markierten Liganden oder an einer erhöhten oder verringerten Umsetzung des markierten Substrates. Moleküle, die gut binden und zu einer erhöhten Aktivität der erfindungsgemäßen Polypeptide führen, sind Agonisten. Moleküle, die gut binden, und die biologische Aktivität der erfindungsgemäßen Polypeptide hemmen, sind gute Antagonisten. Es kann sich dabei auch um Hemmstoffe der oben genannten fungiziden Stoffklasse handeln, jedoch können auch völlige neue Stoffklassen diese modulatorische Aktivität aufweisen. Insbesondere interessant ist die Identifizierung von Inhibitoren der Aldolase, was mit dem beschriebenen Verfahren erzielt werden kann. In diesem Fall kann eine Kandidatenverbindung in einem vor- und nachstehend beschriebenen Test durch eine Hemmung der biologischen Aktivität der Aldolase erkannt werden (Hemmtest).

Die Detektion der biologischen Aktivität der erfindungsgemäßen Polypeptide kann durch ein so genanntes Reportersystem verbessert werden. Reportersysteme in dieser Hinsicht umfassen, sind aber nicht beschränkt auf colorimetrisch oder fluorimetrische Substrate, die in ein Produkt umgewandelt werden oder ein Reportergen, das auf Veränderungen der Aktivität oder der Expression der erfindungsgemäßen Polypeptide anspricht oder andere bekannte Bindungstests.

Ein weiteres Beispiel für ein Verfahren, mit welchem Modulatoren der erfindungsgemäßen Polypeptide aufgefunden werden können, ist ein Verdrängungstest, bei dem man unter dafür geeigneten Bedingungen die erfindungsgemäßen Polypeptide und einen potenziellen Modulator mit einem Molekül, das bekanntermaßen an die erfindungsgemäßen Polypeptide bindet, wie einem natürlichen Substrat oder Liganden oder einem Substrat- oder Liganden-Mimetikum zusammenbringt. Die erfindungsgemäßen Polypeptide selbst können markiert werden, z.B. fluorimetrisch oder colorimetrisch, so dass man die Anzahl der Polypeptide, die an einen Liganden gebunden sind oder die eine Umsetzung mitgemacht haben, exakt bestimmen kann. Auf diese Weise lässt sich die Effektivität eines Agonisten oder Antagonisten ermessen.

Effekte wie Zelltoxizität werden in diesen *in vitro* Systemen in der Regel ignoriert. Die Testsysteme überprüfen

dabei sowohl inhibierende bzw. suppressive Effekte der Substanzen, als auch stimulatorische Effekte. Die Effektivität einer Substanz kann durch konzentrationsabhängige Testreihen überprüft werden. Kontrollansätze ohne Testsubstanzen können zur Bewertung der Effekte herangezogen werden, wobei die gegebenenfalls identifizierte Substanz bezüglich ihrer schädigenden oder abtötenden Wirkung gegen einen oder mehrere Pilze *in vivo* überprüft wird.

Durch die anhand der vorliegenden Erfindung verfügbaren, die für Fructose-1,6-bisphosphat Aldolase kodierende Nukleinsäuren enthaltenden Wirtszellen, aber auch durch die anhand der vorliegenden Erfindung identifizierbaren entsprechenden, homologen Fructose-1,6-bisphosphat Aldolasen aus anderen Spezies, wird die Entwicklung von Testsystemen, die auf Zellen basieren, zur Identifizierung von Substanzen ermöglicht, die die Aktivität der erfindungsgemäßen Polypeptide modulieren.

Eine andere Möglichkeit zur Identifizierung von Substanzen, die die Aktivtät der erfindungsgemäßen Polypeptide modulieren, ist so auch der sogenannten "Scintillation Proximity Assay" (SPA), siehe EP 015 473. Dieses Testsystem nutzt die Interaktion eines Polypeptids (z.B. Fructose-1,6-bisphosphat Aldolase aus *U. maydis*) mit einem radiomarkierten Liganden (z.B. ein kleines organisches Molekül oder ein zweites, radioaktiv markiertes Proteinmolekül). Das Polypeptid ist dabei an kleine Kügelchen ("Microspheres") oder Perlen ("Beads") gebunden, die mit szintillierenden Molekülen versehen sind. Im Verlauf des Abfalls der Radioaktivität wird die szintillierende Substanz im Kügelchen durch die subatomaren Partikel des radioaktiven Markers angeregt und ein detektierbares Photon emittiert. Die Testbedingungen werden so optimiert, dass nur jene vom Liganden ausgehenden Partikel zu einem Signal führen, die von einem an das erfindungsgemäße Polypeptid gebundenen Liganden ausgehen.

In einer möglichen Ausführungsform ist Fructose-1,6-bisphosphat Aldolase aus *U. maydis* an die Beads gebunden, entweder zusammen mit oder ohne interagierende bzw. bindende Testsubstanzen. Verwendet werden könnten dabei auch Fragmente des erfindungsgemäßen Polypeptids. Ein radioaktiv markierter Ligand könnte z. B. ein markiertes, nicht spaltbares Fructose-1,6-bisphosphat-Analog sein. Bei einer Bindung eines bindenden Liganden an die immobilisierte Fructose-1,6-bisphosphat Aldolase, müsste dieser Ligand eine bestehende Interaktion zwischen der immobilisiertem Fructose-1,6-bisphosphat Aldolase und dem markierten Liganden inhibieren oder aufheben, um selbst im Bereich der Kontaktfläche zu binden. Eine erfolgte Bindung an die immobilisierte Fructose-1,6-bisphosphat Aldolase kann dann anhand eines Lichtblitzes detektiert werden. Entsprechend wird ein bestehender Komplex zwischen einem immobilisierten und einem freien, markierten Liganden durch die Bindung einer Testsubstanz zerstört, was zu einem Abfall der detektierten Lichtblitzintensität führt. Das Testsystem entspricht dann einem komplementären Inhibitions-System.

Der Ausdruck "Kompetitor" wie er hierin verwendet wird, bezieht sich auf die Eigenschaft der Verbindungen, mit anderen, gegebenenfalls noch zu identifizierenden Verbindungen um die Bindung an der Aldolase zu kompetitieren und diese vom Enzym zu verdrängen bzw. von dieser verdrängt zu werden.

Der Ausdruck "Agonist", wie er hierin verwendet wird, bezieht sich auf ein Molekül, das die Aktivität der Aldolase beschleunigt oder verstärkt.

Der Ausdruck "Antagonist", wie er hierin verwendet wird, bezieht sich auf ein Molekül, das die Aktivität der Aldolase verlangsamt oder verhindert.

Der Ausdruck "Modulator", wie er hierin verwendet wird, stellt den Oberbegriff zu Agonist bzw. Antagonist dar. Modulatoren können kleine organisch-chemische Moleküle, Peptide oder Antikörper sein, die an die erfindungsgemäßen Polypeptide binden. Weiterhin können Modulatoren kleine organisch-chemische Moleküle, Peptide oder Antikörper sein, die an ein Molekül binden, welches wiederum an die erfindungsgemäßen Polypeptide bindet, und dadurch deren biologische Aktivität beeinflusst. Modulatoren können natürliche Substrate und Liganden darstellen oder strukturelle oder funktionelle Mimetika davon.

Der Ausdruck "Fungizid" bzw. "fungizid", wie er hierin verwendet wird, stellt den Oberbegriff zu Substanzen zur Bekämpfung pflanzenschädigender Pilze und zu Substanzen zur Bekämpfung humanpathogener oder tierpathogener Pilze dar. Der Begriff umfasst damit auch Substanzen, die als Antimycotica verwendet werden können. In einer bevorzugten Bedeutung bezieht sich der Begriff auf Substanzen zur Bekämpfung pflanzenschädigender Pilze.

Vorzugsweise handelt es sich bei den Modulatoren um kleine organisch-chemische Verbindungen.

Die Bindung der Modulatoren an die Aldolase kann die zellulären Vorgänge auf eine Weise verändern, die zum Absterben der damit behandelten phytopathogenen Pilzen führt.

Gegenstand der vorliegenden Erfindung sind deshalb auch Modulatoren von Fructose-1,6-Bisphosphat Aldolasen aus Pilzen, bevorzugt aus phytopathogenen Pilzen, die mit Hilfe eines der in der vorliegenden Anmeldung beschriebenen Verfahrens zum Identifizieren von Modulatoren der Aldolase gefunden werden.

Weiterhin umfasst die vorliegende Erfindung Verfahren zum Auffinden von chemischen Verbindungen, welche die Expression der erfindungsgemäßen Polypeptide verändern. Auch solche "Expressionsmodulatoren" können neue fungizide Wirkstoffe darstellen. Expressionsmodulatoren können kleine organisch-chemische Moleküle, Peptide oder Antikörper sein, die an die regulatorischen Regionen der für die erfindungsgemäßen Polypeptide kodierenden Nukleinsäuren binden. Weiterhin können Expressionsmodulatoren kleine organisch-chemische Moleküle, Peptide oder Antikörper sein, die an ein Molekül binden, welches wiederum an regulatorische Regionen der für die erfindungsgemäßen

Polypeptide kodierenden Nukleinsäuren bindet, und dadurch deren Expression beeinflusst. Expressionsmodulatoren können auch Antisense-Moleküle sein.

Die vorliegende Erfindung bezieht sich ebenfalls auf die Verwendung von Modulatoren der erfindungsgemäßen Polypeptide oder von Expressionsmodulatoren als Fungizide.

Gegenstand der vorliegenden Erfindung sind ebenfalls Expressionsmodulatoren von Fructose-1,6-Bisphosphat Aldolasen, die mit Hilfe des vorstehend beschriebenen Verfahrens zum Auffinden von Expressionsmodulatoren gefunden werden.

Die erfindungsgemäßen Verfahren schließen Hochdurchsatz-Screening (high throughput screening; HTS) und Ultra-Hochdurchsatz-Screening (UHTS) ein. Dafür können sowohl Wirtszellen als auch zellfreie Präparationen verwendet werden, die die erfindungsgemäßen Nukleinsäuren und/oder die erfindungsgemäßen Polypeptide enthalten.

Gegenstand der Erfindung sind weiterhin Antikörper, die spezifisch an die erfindungsgemäßen Polypeptide oder Fragmente davon binden. Die Herstellung solcher Antikörper erfolgt auf die übliche Weise. Beispielsweise können solche Antikörper produziert werden durch die Injektion eines substantiell immunkompetenten Wirts mit einer für die Antikörperproduktion effektiven Menge eines erfindungsgemäßen Polypeptids oder eines Fragments davon und durch nachfolgende Gewinnung dieses Antikörpers. Weiterhin lässt sich in an sich bekannter Weise eine immortalisierte Zelllinie erhalten, die monoklonale Antikörper produziert. Die Antikörper können gegebenenfalls mit einem Nachweisreagenz markiert sein. Bevorzugte Beispiele für ein solches Nachweisreagenz sind Enzyme, radioaktiv markierte Elemente, fluoreszierende Chemikalien oder Biotin. Anstelle des vollständigen Antikörpers können auch Fragmente eingesetzt werden, die die gewünschten spezifischen Bindungseigenschaften zeigen.

Die erfindungsgemäßen Nukleinsäuren können ebenfalls zur Herstellung transgener Organismen wie Bakterien, Pflanzen oder Pilze, bevorzugt zur Herstellung transgener Pflanzen und Pilze, und besonders bevorzugt zur Herstellung transgener Pilze verwendet werden. Diese können z.B. in Testsystemen eingesetzt werden, die auf einer vom Wildtyp abweichenden Expression der erfindungsgemäßen Polypeptide oder Varianten davon basieren. Ferner fallen hierunter sämtliche transgenen Pflanzen oder Pilze, bei denen durch die Modifikation anderer Gene als der vorstehend beschriebenen oder die Modifikation von Genkontrollsequenzen (z.B. Promotor) eine Veränderung der Expression der erfindungsgemäßen Polypeptide oder deren Varianten eintritt. Die transgenen Organismen sind auch zur (Über)produktion des erfindungsgemäßen Polypeptids von Interesse, wobei z.B. Pilze (z.B. Hefe oder *Ustilago maydis*), die im Vergleich zur ihrer natürlichen Form das erfindungsgemäße Polypeptid vermehrt exprimieren, sich besonders zum Einsatz in Verfahren (gerade auch HTS-Verfahren) zum Identifizieren von Modulatoren des Polypeptids eignen.

Für die Herstellung transgener Pflanzen ist das am weitesten entwickelte Vektorsystem ein Plasmid aus dem Bakterium *Agrobacterium tumefaciens.* In der Natur infiziert *A. tumefaciens* Pflanzen und erzeugt Tumoren, die man als Wurzelhalsgallen bezeichnet. Diese Tumoren werden durch das Ti-Plasmid (tumor- inducing) von *A. tumefaciens* hervorgerufen. Das Ti-Plasmid baut ein Stück seiner DNA, das als T-DNA bezeichnet wird, in die chromosomale DNA der Wirtspflanze ein. Es wurde eine Möglichkeit entwickelt, die tumorinduzierenden Bereiche aus der DNA des Plasmids zu entfernen, aber seine Eigenschaft, genetisches Material in die Pflanzen einzubringen, beizubehalten. Dann kann mit Hilfe üblicher rekombinanter DNA-Techniken ein fremdes Gen, z.B. eine der erfindungsgemäßen Nukleinsäuren oder ein erfindungsgemäßes Konstrukt in das Ti-Plasmid eingebaut werden. Das rekombinante Plasmid wird dann in *A. tumefaciens* wieder eingepflanzt, welches dann benutzt werden kann, um eine Pflanzenzellkultur zu infizieren. Man kann das Plasmid aber auch direkt in die Pflanzen einführen, wo es sich in die Chromosomen einbaut. Durch Regeneration solcher Zellen zu intakten Organismen bekommt man Pflanzen, die das Fremdgen enthalten und dieses auch exprimieren, d.h. das erwünschte Genprodukt produzieren.

*A. tumefaciens* infiziert dikotyle Pflanzen zwar leicht, aber für die Transformation von monokotylen Pflanzen, zu denen viele landwirtschaftlich wichtige Kulturpflanzen gehören, wie Mais, Weizen, Reis, ist es als Vektor von beschränktem Nutzen, da es diese nicht ohne weiteres infiziert. Für die Transformation solcher Pflanzen stehen andere Techniken zur Verfügung, wie z.B. "DNA-Kanonen", das so genannte "particle gun" Verfahren. Bei diesem werden kleinste Kügelchen aus Titan oder Gold in Empfängerzellen oder -gewebe geschossen, entweder durch eine Gasentladung oder durch eine Pulverexplosion. Die Kügelchen sind mit DNA der interessierenden Gene beschichtet, wodurch diese in die Zellen gelangen, allmählich abgelöst und in das Genom der Wirtszellen eingebaut werden.

Nur wenige der Zellen, die dem fremden Erbmaterial ausgesetzt werden, sind in der Lage, dieses stabil in ihr eigenes Erbgut einzugliedern. In einem Gewebe, das für den Gentransfer verwendet wird, sind die nicht transgenen Zellen in der Überzahl. Während der Regeneration zur ganzen Pflanze ist es deshalb nötig, eine Selektion anzuwenden, welche die transgenen Zellen bevorteilt. In der Praxis verwendet man dafür Markergene, die in die Pflanzenzellen übertragen werden. Die Produkte dieser Gene inaktivieren einen Hemmstoff, etwa ein Antibiotikum oder Herbizid, und ermöglichen so den transgenen Zellen das Wachstum auf dem mit dem Hemmstoff versetzten Nährmedium. Weniger problematisch sind aber Gene, die für ein Enzym kodieren, das dann nachgewiesen werden kann. Dazu gehört auch das erfindungsgemäße Polypetid, dessen enzymatische Aktivität wie in Beispiel 2 beschrieben nachgewiesen werden

kann.

Bei der Transformation mit *A. tumefaciens* können anstelle von Blattstückchen auch Protoplasten (isolierte Zellen ohne Zellwand, die in Kultur bei Anwesenheit bestimmter Chemikalien oder auch bei Elektroporation Fremd-DNA aufnehmen) benutzen. Man hält sie solange in Gewebekultur, bis sie eine neue Zellwand gebildet haben (z.B. bei Tabak etwa 2 Tage). Dann gibt man Agrobakterien zu und setzt die Gewebekultur fort. Eine einfache Methode zur transienten Transformation von Protoplasten mit einem DNA-Konstrukt ist die Inkubation in Gegenwart von Polyethylenglykol (PEG 4000).

Das Einbringen von DNS in Zellen ist auch durch Elektroporation möglich. Dies ist eine physikalische Methode zur Steigerung der DNS-Aufnahme in lebende Zellen. Durch elektrische Impulse lässt sich die Durchlässigkeit einer Biomembran kurzfristig erhöhen, ohne dass die Membran zerstört wird.

Das Einbringen von DNS ist ebenfalls durch Mikroinjektion möglich. Mit Hilfe von Glaskapillaren wird DNA in die Nähe des Zellkerns einer Zelle injiziert. Dieses ist bei pflanzlichen Zellen, die eine feste Zellwand und eine große Vakuole besitzen, allerdings schwierig.

Eine weitere Möglichkeit beruht auf der Nutzung von Ultraschall: Beschallt man Zellen mit Schallwellen oberhalb des menschlichen Hörbereichs (über 20 kHz), so wird ebenfalls vorübergehende Durchlässigkeit der Membranen beobachtet. Bei dieser Methode muss die Amplitude der Schallwellen sehr fein justiert werden, da die beschallten Zellen sonst platzen und zerstört werden.

Transgene Pilze können in dem Fachmann an sich bekannter Weise hergestellt werden (siehe auch Beispiele).

Gegenstand der Erfindung sind somit auch transgene Pflanzen oder Pilze, die zumindest eine der erfindungsgemäßen Nukleinsäuren enthalten, vorzugsweise transgene Pflanzen wie Arabidopsis species oder transgene Pilze wie Hefe species oder Ustilago species sowie deren transgene Nachkommen. Davon umfasst sind auch die Pflanzenteile, Protoplasten, Pflanzengewebe oder Pflanzenvermehrungsmaterialien der transgenen Pflanzen bzw. die Einzelzellen, Pilzgewebe, Fruchtkörper, Myzel und Sporen der transgenen Pilze, die die erfindungsgemäßen Nukleinsäuren enthalten. Vorzugsweise enthalten die transgenen Pflanzen oder Pilze die erfindungsgemäßen Polypeptide in einer vom Wildtyp abweichenden Form. Als erfindungsgemäß werden jedoch auch solche transgenen Pflanzen oder Pilze betrachtet, die natürlicherweise durch eine nur sehr geringe oder keine Expression des erfindungsgemäßen Polypeptids gekennzeichnet sind.

Gegenstand der vorliegenden Erfindung sind demnach ebenfalls transgene Pflanzen und Pilze, in denen Veränderungen an der für Polypeptide mit der Aktivität einer Fructose-1,6-Bisphosphat Aldolase kodierenden Sequenz vorgenommen wurden und die dann mit Blick auf die Eignung zur Herstellung des erfindungsgemäßen Polypeptids und/oder eine durch Mutagenese erzielte Erhöhung oder Verminderung der biologischen Aktivität oder der Menge des in den Pflanzen oder Pilzen vorliegenden erfindungsgemäßen Polypeptids selektioniert wurden.

Der Ausdruck "Mutagenese" wie er hierin verwendet wird, bezeichnet eine Methode zur Erhöhung der spontanen Mutationsrate und damit zur Isolierung von Mutanten. Dabei können Mutanten mit Hilfe von Mutagenen *in vivo* erzeugt werden, z. B. mit chemischen Verbindungen oder physikalischen Einflüssen, die geeignet sind, Mutationen auszulösen (z.B. Basenanaloga, UV-Strahlen etc.). Die gewünschten Mutanten können durch Selektion auf einen bestimmten Phänotyp hin erhalten werden. Die Position der Mutationen auf den Chromosomen kann relativ zu anderen, bekannten Mutationen durch Komplementations- und Rekombinationsanalysen bestimmt werden. Mutationen können auch gezielt in chromosomale oder extrachromosomale DNA eingebracht werden (*in vitro*-Mutagenese, site-directed Mutagenese oder error-prone-PCR etc.).

Der Ausdruck "Mutante", wie er hierin verwendet wird, bezeichnet einen Organismus, der ein verändertes (mutiertes) Gen trägt. Eine Mutante ist definiert durch den Vergleich mit dem Wildtyp, der das unveränderte Gen trägt.

Die nachfolgenden Beispiele zeigen nun, dass die Aldolase überraschenderweise ein essentielles Enzym in phytopathogenen Pilzen ist, und zeigen weiter, dass das Enzym ein geeignetes Zielprotein für die Identifizierung von Fungiziden ist, in Verfahren zum Identifizieren von fungizid wirksamen Verbindungen verwendet werden kann und dass die in entsprechenden Verfahren identifizierten Modulatoren der Aldolase als Fungizide verwendet werden können.

Weiterhin wird beispielhaft die Gewinnung dieses Enzyms aus *Ustilago maydis* beschrieben und schließlich die Anwendbarkeit der vorliegenden Erfindung für die Suche nach fungizid wirksamen Verbindungen demonstriert.

Die folgenden Beispiele sind nicht beschränkt auf *Ustilago maydis.* Analoge Verfahren und Ergebnisse werden auch in Zusammenhang mit anderen phytopathogenen Pilzen erhalten.

## Beispiele

### Beispiel 1

**Funktionale Expression des Fructose-1,6-bisphosphat Aldolase Gens aus *Ustilago maydis* in *E. coli***

Die kodierende Sequenz der Fructose-1,6-bisphosphat Aldolase aus *Ustilago maydis* wurde in den Expressionsvektor pET 15b (Novagen) kloniert und in den Bakterienstamm BL21 (DE3) (Novagen) transformiert.

Es wurde eine Vorkultur aus frischen Transformanten angeimpft und über Nacht bei 37°C und unter Schütteln in LB-Amp (80 µg/ml) inkubiert. Die Hauptkultur wurde am nächsten Morgen in LB-Amp mit der Vorkultur 1: 25 überimpft und bei 37°C unter Schütteln bis OD = 0,7 angezogen. Danach wurde die Expression des Polypeptids durch Zugabe von 2 mM IPTG (Endkonzentration) induziert und für 48 h bei 15°C durchgeführt. Die Zellen wurden durch Zentrifugation für 10 min bei 4°C und 8000 rpm in Beckman JA14-Rotor gewonnen, indem der Überstand verworfen und das Zellpellet in flüssigem Stickstoff eingefroren und bei -80°C gelagert wurde.

Ein Pellet aus 50 ml Kultur wurde in 4 ml Aufschlußpuffer (100 mM Tris-HCl, pH 8,25; 4 mM 2-Mercaptoethanol; 0,3 % (v/v) Protease Inhibitor for His-tagged Proteins (Sigma); 20 % (v/v) Glycerin; 0,1 mM $ZnCl_2$) aufgenommen und durch Ultraschall (Branson Sonifier, Output 7, 30 %, 5 min) auf Eis aufgeschlossen. Zellbestandteile wurden durch Zentrifugation für 10 min bei 14 000 rpm und 4°C in einem 2 ml-Eppendorf-Gefäß in der Eppendorf-Zentrifuge abgetrennt. Der Überstand, im folgenden Aldolase-Enzympräparation genannt, der das aktive Protein enthält, wurde aliquotiert und bei -20°C gelagert.

### Beispiel 2

**Enzymtest zum Auffinden von Modulatoren der Fructose-1,6-bisphosphat** Aldolase

Die zu testenden Substanzen werden in jeweils 5 µl Puffer in einer 384-Mikrotiterplatte in 10 (v/v) % Dimethylsulfoxid vorgelegt. Die Konzentration der Substanzen wurde so bemessen, dass die Endkonzentration der Substanzen im durchgeführten Test 10 µM betrug. Dazu werden 25 µL Substrat- und Hilfsenzym-Lösung (gekühlt bei 4°C) pipettiert. Darin sind 400 mM Tris-HCl, pH 8,25; 400mM KOAc; je 1,5 µl/ml Glycerin-3-Phosphat-Dehydrogenase/ Triose-Phosphat-Isomerase und Lactat-Dehydrogenase (Hilfsenzyme, alle von Roche Biochemicals) und 6 mM Fructose-1,6-bisphosphat (Substrat) enthalten. Zu diesem Ansatz wurden 25 µL einer verdünnten Aldolase-Enzympräparation gegeben (siehe Bsp. 1). Es wurden dabei circa 100 -500 ng Gesamtprotein eingesetzt. Die Reaktion wurde durch Zugabe von 45 µl NADH (533 µM in 5 mM Tris-HCl, pH 8,25) (gekühlt bei 4°C) gestartet. Es wurde die Abnahme der Fluoreszenz bei λ= 360/35 nm (Exc) und λ=465/35 nm (Em) bei Raumtemperatur für 30-45 min gemessen (wird in Vormessung ermittelt), wobei die Ergebnisse einer Messung bei Anwesenheit einer zu testenden Verbindung mit den Ergebnissen einer Messung bei Abwesenheit einer zu testenden Verbindung verglichen wurden.

Die im Test verwendeten Substanzen lagen in folgenden Endkonzentrationen vor bzw. besaßen die folgende Aktivität:

c(Tris-HCl) = 125 mM, c(KOAc) = 100 mM, c(MESH) = 1 mM, c(P-Inhibitor) = 0,075 %, (v/v), c(Glycerin) = 5 % (v/v), c($ZnCl_2$) = 20 µM, c(F-1,6BP) = 1,5 mM, c(NADH) = 240µM, Akt. (GDH) = 0,0124 U, Akt. (TIM) = 0,0581 U, Akt. (LDH) = 0,103 U, m(Aldolase) = 100-500 ng.

**SEQ ID NO:1:** Genomische Sequenz kodierend für die Fructose-1,6bisphosphat Aldolase aus *Ustilago maydis*. Die Sequenz aus Ustilago maydis enthält ein Intron. Die kodierenden Bereiche sind erkennbar.

**SEQ ID NO:2:** Aminosäuresequenz kodiert von Exon (I) der genomischen Sequenz gemäß SEQ ID NO:1 kodierend für Fructose-1,6bisphosphat Aldolase aus *Ustilago maydis.*

**SEQ ID NO:3:** Aminosäuresequenz kodiert von Exon (II) der genomischen Sequenz gemäß SEQ ID NO:1 kodierend für Fructose-1,6bisphosphat Aldolase aus *Ustilago maydis.*

**SEQ ID NO:4:** cDNA Sequenz kodierend für die Fructose-1,6-bisphosphat Aldolase aus *Ustilago maydis.*

**SEQ ID NO: 5:** Aminosäuresequenz kodiert von der cDNA Sequenz gemäß SEQ ID NO:4 kodierend für Fructose-1,6-bisphosphat Aldolase aus *Ustilago maydis.*

**Abbildung 1:** Multiples Alignment der Sequenzen aus Tabelle 1 mit dem Programm Pileup mit GCG aus dem Wisconsin Package, Version 10.2, 1998-2001 (Gap Creation Penalty: 8, Gap Extension Penalty: 2).

Die Konsensussequenz wurde bestimmt mit dem Programm Pretty aus dem GCG-Package (Minimum Plurality: 5)

## Literatur

Altschul, S.F., Madden, T.L., Schaffer, A.A., Zhang, J.Z.; Miller W. and Lipman, D.J. (1997). Gapped BLAST und PSI-BLAST generation of protein database search programs. Nucleic Acids Res. 25: 3389-3402.

Gamblin, S. J. (1991): Activity and Specificity of Human Aldolases J. Mol. Biol. 219:573-576.

Hall D.R. et al. (1999): The crystal structure of Escherichia coli class II fructose-1,6-bisphosphate Aldolase in complex with phosphoglycolohydroxamate reveals details of mechanism and specificity. J. Mol. Biol. 287: 383-394.

Hofmann K., Bucher P., Falquet L., Bairoch A. (1999): The PROSITE database, its status in 1999. Nucleic Acids Res. 27:215-219.

Lottspeich, F., Zorbas H. (Hrsg.). (1998). Bioanalytik. Spektrum Akademischer Verlag, Heidelberg, Berlin.

Marsh J. J. and Lebherz H G (1992): Fructose-bisphosphate aldolases: an evolutionary history. TIBS 17: 110-113.

P. Willnow: "Fructose-1,6-bisphosphate Aldolase" in H. Bergmeyer: Enzymatic Analysis" (1985), pp 346-353, Verlag Chemie.

Schwelberger H. G. et al. (1989): Molecular cloning, primary structure and disruption of the structural gene of Aldolase from Saccharomyces cerevisiae. Eur. J. Biochem. 180: 301-308.

## Patentansprüche

1. Verfahren zum Identifizieren von Fungiziden, **dadurch gekennzeichnet, dass** man ein Kandidatenmolekül in einem Fructose-1,6-bisphosphat Aldolase-Hemmtest prüft und die Kandidatenmoleküle auswählt, die eine hemmende Wirkung auf die Aktivität der Fructose-1,6-bisphosphat Aldolase ausüben.

**2.** Verfahren gemäß Anspruch 1, **dadurch gekennzeichnet, dass** man das ausgewählte Kandidatenmolekül anschließend auf seine fungizide Wirkung in vivo überprüft.

**3.** Verwendung von Fructose-1,6-bisphosphat Aldolase aus Pilzen, von dafür kodierenden Nukleinsäuren, DNA-Konstrukten oder Wirtszellen enthaltend diese Nukleinsäuren zum Auffinden von fungiziden Wirkstoffen.

**4.** Verwendung eines Modulators eines Polypeptids mit der biologischen Aktivität einer Fructose-1,6-bisphosphate Aldolase als Fungizid und/oder Antimycotikum.

**5.** Modulatoren der Fructose-1,6-bisphosphat Aldolase aus Pilzen, welche durch ein Verfahren gemäß Anspruch 1 oder 2 identifiziert werden.

**6.** Nukleinsäure, kodierend für ein Polypeptid mit der Aktivität einer Fructose-1,6-bisphosphat Aldolase aus pflanzenpathogenen Pilzen.

**7.** Nukleinsäure gemäß Anspruch 6, **dadurch gekennzeichnet, dass** sie für Fructose-1,6-bisphosphat Aldolase aus Basidiomyceten kodiert.

**8.** Nukleinsäure gemäß Anspruch 6 oder 7, **dadurch gekennzeichnet, dass** sie für Fructose-1,6-bisphosphat Aldolase aus Ustilago kodiert.

**9.** Nukleinsäuren gemäß Anspruch 6, 7 oder 8, **dadurch gekennzeichnet, dass** es sich um einzelsträngige oder doppelsträngige DNA oder RNA handelt.

**10.** Nukleinsäuren gemäß einem der Ansprüche 6 bis 9, **dadurch gekennzeichnet, dass** es sich um Fragmente genomischer DNA oder um cDNA handelt.

**11.** Nukleinsäuren gemäß einem der Ansprüche 6 bis 10, umfassend eine Sequenz ausgewählt aus

a) der Sequenz gemäß SEQ ID NO: 1 und SEQ ID NO: 4

b) Sequenzen, die für ein Polypeptid codieren, welches die Aminosäuresequenz gemäß SEQ ID NO: 2 und 3 oder SEQ ID NO: 5 umfasst,

c) Sequenzen, die für ein Polypeptid kodieren, welches die Konsensusmotive -(YGIPVV)-x-(LHTDHC)- und -(LEMEIGITGGEEDGV)- umfassen,

d) zumindest 14 Basenpaare langen Teilsequenzen der unter a) bis c) definierten Sequenzen,

e) Sequenzen, welche an die unter a) bis c) definierten Sequenzen bei einer Hybridisierungstemperatur von 35-52°C hybridisieren,

f) Sequenzen, welche eine zumindest eine 60 %ige, bevorzugt eine 80 %ige, besonders bevorzugt eine 90 %ige und ganz besonders bevorzugt eine 95 %ige Identität mit den unter a) bis c) definierten Sequenzen aufweisen,

g) Sequenzen, welche zu den unter a) bis c) definierten Sequenzen komplementär sind, und

h) Sequenzen, welche aufgrund der Degeneriertheit des genetischen Codes für dieselbe Aminosäuresequenz codieren wie die unter a) bis f) definierten Sequenzen.

**12.** DNA-Konstrukt umfassend eine Nukleinsäure gemäß einem der Ansprüche 6 bis 11 und einen heterologen Promotor.

**13.** Vektor umfassend eine Nukleinsäure gemäß einem der Ansprüche 6 bis 11, oder ein DNA-Konstrukt gemäß Anspruch 12.

**14.** Vektor gemäß Anspruch 13, **dadurch gekennzeichnet, dass** die Nukleinsäure funktionell mit regulatorischen Sequenzen verknüpft ist, die die Expression der Nukleinsäure in pro- oder eukaryotischen Zellen gewährleisten.

**15.** Wirtszelle enthaltend eine Nukleinsäure gemäß einem der Ansprüche 6 bis 11, ein DNA-Konstrukt gemäß Anspruch 12 oder einen Vektor gemäß Anspruch 13 oder 14.

**16.** Wirtszelle gemäß Anspruch 15, **dadurch gekennzeichnet, dass** es sich um eine prokaryotische Zelle handelt.

**17.** Wirtszelle gemäß Anspruch 16, **dadurch gekennzeichnet, dass** es sich um eine eukaryotische Zelle handelt.

**18.** Polypeptid mit der biologischen Aktivität einer Fructose-1,6-bisphosphat Aldolase, welches von einer Nukleinsäure gemäß einem der Ansprüche 6 bis 11 kodiert wird.

**19.** Polypeptid mit der biologischen Aktivität einer Fructose-1,6-bisphosphat Aldolase, welches eine Aminosäuresequenz umfasst, die eine zumindest 60 %ige Ähnlichkeit mit der Sequenz gemäß SEQ ID NO: 2 und 3 oder SEQ ID NO:5 aufweist.

**20.** Antikörper, welcher spezifisch an ein Polypeptid gemäß Anspruch 18 oder 19 bindet.

**21.** Verfahren zum Herstellen einer Nukleinsäure gemäß einem der Ansprüche 6 bis 11, umfassend die folgenden Schritte:

a) Vollständige chemische Synthese auf an sich bekannte Weise oder

(b) chemische Synthese von Oligonukleotiden, Markieren der Oligonukleotide, Hybridisieren der Oligonukleotide an DNA einer genomischen oder cDNA-Bank, die ausgehend von genomischer DNA bzw. mRNA aus Pilzzellen hergestellt wurde, Selektieren von positiven Klonen und Isolieren der hybridisierenden DNA aus positiven Klonen oder

(c) chemische Synthese von Oligonukleotiden und Amplifizierung der Ziel-DNA mittels PCR.

**22.** Verfahren zum Herstellen eines Polypeptids gemäß Anspruch 18 oder 19, umfassend

a) das Kultivieren einer Wirtszelle gemäß einem der Ansprüche 15 bis 17 unter Bedingungen, die die Expression der Nukleinsäure gemäß einem der Ansprüche 6 bis 11 gewährleisten, oder

b) das Exprimieren einer Nukleinsäure gemäß einem der Ansprüche 6 bis 11 in einem *in vitro*-System, und

(c) die Gewinnung des Polypeptids aus der Zelle, dem Kulturmedium oder dem *in vitro*-System.

**23.** Verfahren zum Auffinden einer Verbindung, welche die Expression von Polypeptiden gemäß Anspruch 18 oder 19 verändert, umfassend die folgenden Schritte:

a) Inkontaktbringen einer Wirtszelle gemäß einem der Ansprüche 15 bis 17 mit einer chemischen Verbindung oder einem Gemisch von chemischen Verbindungen,

b) Bestimmen der Polypeptidkonzentration, und

c) Bestimmen der Verbindung, welche die Expression des Polypeptids spezifisch beeinflusst.

**24.** Verwendung einer Nukleinsäure gemäß einem der Ansprüche 6 bis 11, eines DNA-Konstrukts gemäß Anspruch 12 oder eines Vektors gemäß Anspruch 13 oder 14 zum Herstellen von transgenen Pflanzen und Pilzen.

**25.** Transgene Pflanzen, Pflanzenteile, Protoplasten, Pflanzengewebe oder Pflanzenvermehrungsmaterialien, **dadurch gekennzeichnet, dass** nach Einbringen einer Nukleinsäure gemäß einem der Ansprüche 6 bis 11, eines DNA-Konstrukts gemäß Anspruch 12 oder eines Vektors gemäß Anspruch 13 oder 14 die intrazelluläre Konzentration eines Polypeptids gemäß Anspruch 18 oder 19 im Vergleich zu den entsprechenden Wildtyp-Zellen erhöht oder vermindert ist.

**26.** Transgene Pilze, Pilzzellen, Pilzgewebe, Fruchtkörper, Mycele und Sporen, **dadurch gekennzeichnet, dass** nach Einbringen einer Nukleinsäure gemäß einem der Ansprüche 6 bis 11, eines DNA-Konstrukts gemäß Anspruch 12 oder eines Vektors gemäß Anspruch 13 oder 14 die intrazelluläre Konzentration eines Polypeptids gemäß Anspruch 18 oder 19 im Vergleich zu den entsprechenden Wildtyp-Zellen erhöht oder vermindert ist.

**27.** Pflanzen, Pflanzenteile, Protoplasten, Pflanzengewebe oder Pflanzenvermehrungsmaterialien, **dadurch gekennzeichnet, dass** sie ein Polypeptid gemäß Anspruch 18 oder 19 enthalten, dessen biologische Aktivität oder Expressionsmuster im Vergleich zu den entsprechenden endogenen Polypeptiden verändert ist.

**28.** Verfahren zum Herstellen von Pflanzen, Pflanzenteilen, Protoplasten, Pflanzengeweben oder Pflanzenvermehrungsmaterialien gemäß Anspruch 27, **dadurch gekennzeichnet, dass** man eine Nukleinsäure gemäß einem der Ansprüche 6 bis 11 durch endogene Mutagenese verändert.

**29.** Pilze, Pilzzellen, Pilzgewebe, Fruchtkörper, Mycele und Sporen, **dadurch gekennzeichnet, dass** sie ein Polypeptid gemäß Anspruch 18 oder 19 enthalten, dessen biologische Aktivität oder Expressionsmuster im Vergleich zu den entsprechenden endogenen Polypeptiden verändert ist.

**30.** Verfahren zum Herstellen von Pilzen, Pilzzellen, Pilzgewebe, Mycelen und Sporen gemäß Anspruch 29, **dadurch gekennzeichnet, dass** man eine Nukleinsäure gemäß einem der Ansprüche 6 bis 11 durch endogene Mutagenese verändert.

```
B. graminis      ~~~~~~~THY LNIFNQNTHL ISQSEKMGAM ..DVLSRKSG VIVGDDVLKL
C. immitis       ~~~~~~~~~~ ~~~~~~~~~~ ~~~~~~MGVV ..DALSRKSG VIVGDDVLRL
C. fulvum        HHSTCIHSPY *VIW*KLTSH HNHNRITATM AQSVLKXKSG VIYGDDVKNL
K. lactis        ~~~~~~~~~~ ~~~~~~~~~~ ~~~~~~~~~~ ~~~~~~~~~~ ~~~~~~~~~~
S. cerevisiae    ~~~~~~~~~~ ~~~~~~~~~~ ~~~~~~~~~G VEQILKRKTG VIVGEDVHNL
U. maydis        ~~~~~~~~~~ ~~~~~~~~~~ ~~~~~~~~~~ MGVLDIVPAG VVSGKDVYKV
S. pombe         ~~~~~~~~~~ ~~~~~~~~~~ ~~~~~~~~~~ MGILDIVPTG VIAGDNVLKL
N. crassa        ~~~~~~~~~~ ~~~~~~~~~~ ~~~~~MRCPI PEHKQNLTFS LPLGDDVLKL
A. oryzae        ~~~~~~~~~~ ~~~~~~~~~~ ~~~~~~MGVG VLEKLSRKTG VIVGDDVLRL
M. graminicola   ~~~~~~~~~~ ~~~~~~~~~~ ~~~~~~~~~~ ~~~~~~~~~~ ~~~~~~~~~~
C. neoformans    ~~~~~~~~~~ ~~~~~~~~~~ ~~~~~~~~~~ ~~~~~~~~~~ ~~~~~~~~~~
Consensus        ---------- ---------- ---------- ----L--K-G VI-GDDVL-L

                 51                                                100
B. graminis      FQYAKDHKFA IPAINVTSSS TVVASLEAAR DAKAPIVLQM SQGGAAYFAG
C. immitis       FEYAQQNNFA IPAVNVTSSS TAVAALEAAR DKKAPIILQM SQGGAAYFAG
C. fulvum        FKYAQXKNFA IPAINVTSSS TVVASLEAAR DAKAPLILQF SQGGAAYFAG
K. lactis        ~~~~~~~~~~ ~~~~~~~~~~ ~~~~~~~~AR DNKSPIILQT SNGGAAYFAG
S. cerevisiae    FTYAKEHKFA IPAINVTSSS TAVAALEAAR DSKSPIILQT SNGGAAYFAG
U. maydis        FDYARQNKFA IPAFNVTSSS VAISALEAAR DAKSPLILQV SQGGAANFAG
S. pombe         FTYAREHGFA IPAINVTSSS TAIAALEAAR EARSPIILQT SNGGAHFFAG
N. crassa        FQYAREKQFA IPACNVTSSS TAVAALEAAR DQKAPSSLQT SQGCAAFFAG
A. oryzae        FEHAQQNNYA IPAVNVTSSS TVVASLEAAR DQNCPIVLQL SQGGAAYFAG
M. graminicola   ~~~~~~~~~~ ~~~~~~~~~~ ~~~~~~~~~~ ~~~~~~~~~~ ~~~~~~~~~~
C. neoformans    ~~~~~~~~~~ ~~~~~~~~~~ ~~~~~~~~~~ ~~~~~~~~~~ ~~~~~~~~~~
Consensus        F-YA----FA IPAINVTSSS TAVAALEAAR D-K-PIILQ- SQGGAAYFAG

                 101                                               150
B. graminis      KGVANGGQEA ..SIAGGIAG AHYIRAIAPA YGIPVI.LHT DHCAKKLLPW
C. immitis       KGVTNTNQ.A ..SIAGAVAG AHYIRSVAPI YAIPVV.LHT DHCAKKLLPW
C. fulvum        KGVDNKDQSA ..SIQGXIAA THYVRAIAPA YGIPVV.LHT DHCAKKLLPW
K. lactis        KGVSNEGQNA ..SIRGSIAA AHYIRSIAPA YGIPVV.LHT DHCAKKLLPW
S. cerevisiae    KGISNEGQNA ..SIKGAIAA AHYIRSIAPA YGIPVV.LHS DHCAKKLLPW
U. maydis        KGLSNSNQEA ..SIIGAKAA ALFIRAVAPS YGVPVI.MHS DHCAKKLLPW
S. pombe         KESSNEGQKA ..SIAGSIAA AHYIRSIAPF FGVPVV.MHS DHCAKKLLPW
N. crassa        KGIKDSAEKA RDSVAGAIAA AHYIRSIAPI YGIPVVRIHT DHCAKKLL.F
A. oryzae        KGVSNDGQQA PLPVVSLLPT TS.VALLPPT VSLLSFTPTT APRSSSLGSM
M. graminicola   ~~~~~~~~~~ ~~~~~~~~~~ ~~~~~~~~PS YGIPVV.LHT DHCAKKLLPW
C. neoformans    ~~~~~~~~~~ ~~~~~~~~~~ ~~~~~~~~~~ ~~~~~~~~~~ ~~~~~~~~~~
Consensus        KGVSN-GQ-A --SI-G-IAA AHYIRSIAP- YGIPVV-LHT DHCAKKLLPW
                                                  Motif 1
                 151                                               200
B. graminis      LDGLLDADEA YFKIHGEPFV STI*L.TYL~ ~~~~~~~~~~ ~~~~~~~~~~
C. immitis       FDGMLDADEK ~~~~~~~~~~ ~~~~~~~~~~ ~~~~~~~~~~ ~~~~~~~~~~
C. fulvum        LDGMLDADEX EFKAXGTPLX SRHDR.FXEE PVKE.NIEXP ASTLARLNNE
K. lactis        FDGMLKADEE YFAKHGEPLF SSHMLDLSEE TDEE.NIGLC VKYFTRMAKI
S. cerevisiae    FDGMLEADEA YFKEHGEPLF SSHMLDLSEE TDEE.NISTC VKYFKRMAAM
U. maydis        FDGMLAADEE YYKEHNEPLF SSHMLDLSEE SKEE.NIETC LKYLKRMAPL
S. pombe         MDGMFEADEA YFKIHGEPLF SSHMLDLSEE PKKE.NIAQV KEYCKRAVPM
N. crassa        GDGIPEEDEK FFKANGVPLF SSHMIDLSEE PVEE.NISTC VKYLKRMAPM
A. oryzae        ASSTRMSATS ..SSTASPFF SSHMIDLSEE PVDY.NIQTT AAYLKRAAPM
M. graminicola   LDGMLDADKA FFKEHGTPLF SSHMIDLSEE TVEEKNLNTT AQYLKRAAPM
C. neoformans    ~~~~~~~~~~ ~~~~~~~~~~ ~~~~~~~~~~ ~~~~~~~~~~ ~~~~~~~~~~
Consensus        -DGML-ADE- YFK-HGEPLF SSHMLDLSEE --EE-NI-T- --YLKR-APM
```

```
               201                                                    250
B. graminis    ~~~~~~~~~~ ~~~~~~~~~~ ~~~~~~~~~~ ~~~~~~~~~~ ~~~~~~~~~~
C. immitis     ~~~~~~~~~~ ~~~~~~~~~~ ~~~~~~~~~~ ~~~~~~~~~~ ~~~~~~~~~~
C. fulvum      XWLER~~~~~ ~~~~~~~~~~ ~~~~~~~~~~ ~~~~~~~~~~ ~~~~~~~~~~
K. lactis      HQWLEMEIGI TGGEEDGVNN EGTSNDKLYT TPETVFSVHE ALSKISPNFS
S. cerevisiae  DQWLEMEIGI TGGEEDGVNN ENADKEDLYT KPEQVYNVYK ALHPISPNFS
U. maydis      GIWLEMEIGI TGGEEDGVNN EGVDNASLYT QPEDIWDIYR QFSEITPNFS
S. pombe       KIWIEMEIGI TGGEEDGVDN SHVSHTELYT QPEDIWDVYR ELSSVTPYFS
N. crassa      KQWLEMEIGI TGGEEDGVDN SEVDNASLYT QPEDIWQIEE DSRPISPYFS
A. oryzae      KQWLEMEIGI TGGEEDGVNN EDVDNNSLYT QPEDILAIHK ALSPISPYFS
M. graminicola KQFLEMEIGI TGGEEDGVNN EDVDNNSLYT QPEDIHNIYK TLAPISPYFS
C. neoformans  ~~~~~~~~~~ ~~~~~~~~~~ ~~~~~~~~~~ ~~~~~~~~~~ ~~~~~~~~~~
Consensus      -QWLEMEIGI TGGEEDGVNN E-VDN--LYT QPEDI--I-- -L--ISP-FS
                  Motif 2
               251                                                    300
B. graminis    ~~~~~~~~~~ ~~~~~~~~~~ ~~~~~~~~~~ ~~~~~~~~~~ ~~~~~~~~~~
C. immitis     ~~~~~~~~~~ ~~~~~~~~~~ ~~~~~~~~~~ ~~~~~~~~~~ ~~~~~~~~~~
C. fulvum      ~~~~~~~~~~ ~~~~~~~~~~ ~~~~~~~~~~ ~~~~~~~~~~ ~~~~~~~~~~
K. lactis      IASAFGNVHG VYK.IAAALK PELLGTFQDY AAKQLNKKAE DKPLYLVFHG
S. cerevisiae  IAAAFGNCHG LYA.GDIALR PEILAEHQKY TREQVGCK.E EKPLFLVFHG
U. maydis      IAAGFGNVHG VYKPGNVSLQ PELLGKHQEH VRAQI.KSDK ENPVFFVFHG
S. pombe       IAAAFGNVHG VYKPGNVKLQ PALLGQHQAY VKEQL.KTTN DKPVFFVFHG
N. crassa      IGAGFGNVHG VYAPGNVKLH PDLLGKQQAY VPEKLGGKDK .KPFFFVFHG
A. oryzae      IAAGFGNVHG VYKPGNVKLH PELLKKHQAY VKEKIG.SNK DKPVFFVFHG
M. graminicola IAAGFGNLHG SLQARQR*AP PRAPPEAPKF .RARAGEDPG GEPVFLVFHG
C. neoformans  ~~~~~~~~~~ ~~~~~~~~~~ ~~~~~~~~~~ ~~~~~~~~~~ ~~~~~~~~~~
Consensus      IAA-FGNVHG VY--G-V-L- P-LL---Q-Y ---------- -KP-F-VFHG

               301                                                    350
B. graminis    ~~~~~~~~~~ ~~~~~~~~~~ ~~~~~~~~~~ ~~~~~~~~~~ ~~~~~~~~~~
C. immitis     ~~~~~~~~~~ ~~~~~~~~~~ ~~~~~~~~~~ ~~~~~~~~~~ ~~~~~~~~~~
C. fulvum      ~~~~~~~~~~ ~~~~~~~~~~ ~~~~~~~~~~ ~~~~~~~~~~ ~~~~~~~~~~
K. lactis      GSGSSTKDFH TAIDFGVVKV NLDTDCQFAY LSGIRDYVLN KKDY~~~~~~
S. cerevisiae  GSGSTVQEFH TGIDNGVVKV NLDTDCQYAY LTGIRDYVLN KKDYIMSPVG
U. maydis      GSGSEKHEIS TAVSHGVVKM NVDTDT.WAY LLGIRDYILN KKDYLMSQVG
S. pombe       GSGSSVNEFR TGIKCGVVKV NIDTDTQFAY VEGVRDYVLK YKDYLMTPVG
N. crassa      GSGSSKEEYR EAISNGVVKV NVDTDLQWSY LVGIRDYILN NIDYLRSQVG
A. oryzae      GSGSSKEEYK EAISYGVVKV NVDTDMQFAY MSGIRDYILK KKDYLMTAVG
M. graminicola GSGSXKQDYL DPITTVSSRS TSTPICNTAY ~~~~~~~~~~ ~~~~~~~~~~
C. neoformans  ~~~~~~~~~~ ~~~~~~~~~~ ~~~~~~~~~~ ~~~~~~~~~~ ~~~~~~~~~~
Consensus      GSGS---E-- --I--GVVKV N-DTD-Q-AY --GIRDY-L- -KDY----VG

               351                                                397
B. graminis    ~~~~~~~~~~ ~~~~~~~~~~ ~~~~~~~~~~ ~~~~~~~~~~ ~~~~~~~
C. immitis     ~~~~~~~~~~ ~~~~~~~~~~ ~~~~~~~~~~ ~~~~~~~~~~ ~~~~~~~
C. fulvum      ~~~~~~~~~~ ~~~~~~~~~~ ~~~~~~~~~~ ~~~~~~~~~~ ~~~~~~~
K. lactis      ~~~~~~~~~~ ~~~~~~~~~~ ~~~~~~~~~~ ~~~~~~~~~~ ~~~~~~~
S. cerevisiae  NPEGPEKPNK KFFDPRVWVR EGEKTMGAKI TKSLETFRTT NTL~~~~
U. maydis      NPDGADKPNK KFFDPRVWVR EGEKTMATRC KEAFTDLGST GKL~~~~
S. pombe       NPEGADKPNK KKFDPRVWIH EGEKTMTKRV LTALEDFYTV NTL~~~~
N. crassa      NPEGPNKPNK KKYDPRVWIR EGEKTMKARV EEGPQGPSNT TVTV~~~
A. oryzae      NPEGEDKPNK KSFDPRVWVR EGEKTMSQRV KVALEDFNTA GQL~~~~
M. graminicola ~~~~~~~~~~ ~~~~~~~~~~ ~~~~~~~~~~ ~~~~~~~~~~ ~~~~~~~
C. neoformans  ~~~~~~~~NK KQYDPRVWVR EGEKTLVERV KEACVDLXXT TTGTKIG
Consensus      NP-G--KPNK K-FDPRVWVR EGEKTM--RV ---------- -------
```

**Figure 1**

Europäisches Patentamt

# EUROPÄISCHER TEILRECHERCHENBERICHT

der nach Regel 45 des Europäischen Patentübereinkommens für das weitere Verfahren als europäischer Recherchenbericht gilt

Nummer der Anmeldung
EP 02 01 8190

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int.Cl.7) |
|---|---|---|---|
| X | DATABASE EMBL [Online] EMBL; 30. August 2000 (2000-08-30) "fructose 1,6-bisphosphate aldolase" Database accession no. AB032272 XP002219325 * Zusammenfassung * --- | 6,11 | C12Q1/527<br>C12N15/52<br>C12N9/88 |
| A | DATABASE BIOSIS [Online] BIOSCIENCES INFORMATION SERVICE, PHILADELPHIA, PA, US; 1983 UCHIYAMA T ET AL: "LIGNIFICATION AND QUALITATIVE CHANGES OF PHENOLIC COMPOUNDS IN RICE ORYZA-SATIVA CALLUS TISSUES INOCULATED WITH PLANT PATHOGENIC FUNGI" Database accession no. PREV198376036764 XP002219326 * Zusammenfassung * & AGRICULTURAL AND BIOLOGICAL CHEMISTRY, Bd. 47, Nr. 1, 1983, Seiten 1-10, ISSN: 0002-1369 --- -/-- | 6,11 | RECHERCHIERTE SACHGEBIETE (Int.Cl.7)<br>C12N<br>C12Q |

## UNVOLLSTÄNDIGE RECHERCHE

Die Recherchenabteilung ist der Auffassung, daß ein oder mehrere Ansprüche, den Vorschriften des EPÜ in einem solchen Umfang nicht entspricht bzw. entsprechen, daß sinnvolle Ermittlungen über den Stand der Technik für diese Ansprüche nicht, bzw. nur teilweise, möglich sind.

Vollständig recherchierte Patentansprüche:

Unvollständig recherchierte Patentansprüche:

Nicht recherchierte Patentansprüche:

Grund für die Beschränkung der Recherche:

Siehe Ergänzungsblatt C

| Recherchenort | Abschlußdatum der Recherche | Prüfer |
|---|---|---|
| MÜNCHEN | 6. November 2002 | Lüdemann, S |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C09)

Europäisches Patentamt

**UNVOLLSTÄNDIGE RECHERCHE ERGÄNZUNGSBLATT C**

Nummer der Anmeldung
EP 02 01 8190

Vollständig recherchierte Ansprüche:
1-3

Nicht recherchierte Ansprüche:
4,5

Grund für die Beschränkung der Recherche:

Die geltenden Patentansprüche 4 und 5 beziehen sich auf eine unverhältnismäßig große Zahl möglicher Verbindungen. In der Tat umfassen sie so viele Wahlmöglichkeiten, dass sie im Sinne von Art. 84 EPÜ in einem solche Maße unklar oder zu weitläufig gefasst erscheinen, als daß sie eine sinnvolle Recherche ermöglichten. Da ausserdem keine technischen Merkmale für die in Anspruch 4 und 5 beschriebenen Modulatoren angegeben wurden, konnte die Recherche nicht durchgeführt werden.

Europäisches Patentamt

**EUROPÄISCHER TEILRECHERCHENBERICHT**

Nummer der Anmeldung
EP 02 01 8190

| EINSCHLÄGIGE DOKUMENTE | | | KLASSIFIKATION DER ANMELDUNG (Int.Cl.7) |
|---|---|---|---|
| Kategorie | Kennzeichnung des Dokuments mit Angabe, soweit erforderlich der maßgeblichen Teile | Betrifft Anspruch | |
| X | SOLEL Z ET AL: "EFFECT OF THE FUNGICIDES GUAZATINE AND DODINE ON GROWTH AND METABOLISM OF USTILAGO-MAYDIS" ZEITSCHRIFT FUER PFLANZENKRANKHEITEN UND PFLANZENSCHUTZ, Bd. 91, Nr. 3, 1984, Seiten 273-285, XP001118362 ISSN: 0340-8159 Seiten 276, 2.8; 283, 3.7; 284 --- | 1-5 | |
| X | STEHMANN CHRISTIANE ET AL: "Inhibition of enzymes of the glycolytic pathway and hexose monophosphate bypass by phosphonate." PESTICIDE BIOCHEMISTRY AND PHYSIOLOGY, Bd. 67, Nr. 1, Mai 2000 (2000-05), Seiten 13-24, XP002219324 ISSN: 0048-3575 Seiten: 15, Tabelle 3 * Zusammenfassung * --- | 1-5 | RECHERCHIERTE SACHGEBIETE (Int.Cl.7) |
| X | KOWALLIK WOLFGANG ET AL: "Complete sequence of glycolytic enzymes in the mycorrhizal basidiomycete, Suillus bovinus." ZEITSCHRIFT FUER NATURFORSCHUNG SECTION C JOURNAL OF BIOSCIENCES, Bd. 53, Nr. 9-10, 1998, Seiten 818-827, XP001119637 ISSN: 0939-5075 S. 820, rechte Spalte, oben; Tabelle 1,4 ----- | 1-3 | |

EPO FORM 1503 03.82 (P04C12)

Europäisches
Patentamt

Nummer der Anmeldung
EP 02 01 8190

## GEBÜHRENPFLICHTIGE PATENTANSPRÜCHE

Die vorliegende europäische Patentanmeldung enthielt bei ihrer Einreichung mehr als zehn Patentansprüche.

☐ Nur ein Teil der Anspruchsgebühren wurde innerhalb der vorgeschriebenen Frist entrichtet. Der vorliegende europäische Recherchenbericht wurde für die ersten zehn sowie für jene Patentansprüche erstellt, für die Anspruchsgebühren entrichtet wurden, nämlich Patentansprüche:

☐ Keine der Anspruchsgebühren wurde innerhalb der vorgeschriebenen Frist entrichtet. Der vorliegende europäische Recherchenbericht wurde für die ersten zehn Patentansprüche erstellt.

## MANGELNDE EINHEITLICHKEIT DER ERFINDUNG

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

Siehe Ergänzungsblatt B

☐ Alle weiteren Recherchengebühren wurden innerhalb der gesetzten Frist entrichtet. Der vorliegende europäische Recherchenbericht wurde für alle Patentansprüche erstellt.

☐ Da für alle recherchierbaren Ansprüche die Recherche ohne einen Arbeitsaufwand durchgeführt werden konnte, der eine zusätzliche Recherchengebühr gerechtfertigt hätte, hat die Recherchenabteilung nicht zur Zahlung einer solchen Gebühr aufgefordert.

☐ Nur ein Teil der weiteren Recherchengebühren wurde innerhalb der gesetzten Frist entrichtet. Der vorliegende europäische Recherchenbericht wurde für die Teile der Anmeldung erstellt, die sich auf Erfindungen beziehen, für die Recherchengebühren entrichtet worden sind, nämlich Patentansprüche:

☒ Keine der weiteren Recherchengebühren wurde innerhalb der gesetzten Frist entrichtet. Der vorliegende europäische Recherchenbericht wurde für die Teile der Anmeldung erstellt, die sich auf die zuerst in den Patentansprüchen erwähnte Erfindung beziehen, nämlich Patentansprüche:

1-2, 3 (teilweise)

Europäisches Patentamt

**MANGELNDE EINHEITLICHKEIT DER ERFINDUNG ERGÄNZUNGSBLATT B**

Nummer der Anmeldung
EP 02 01 8190

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

1. Ansprüche: 1-2, 3 (teilweise)

   Verfahren zum Identifizieren von Fungiziden, dadurch gekennzeichnet, dass man ein Kandidatenmolekül in einem Fructose-1,6-biphosphat Aldolase-Hemmtest prüft und die Kandidatenmoleküle auswählt, die ein hemmende Wirkung auf die Aktivität der Fructose-biphosphat-aldolase ausüben

2. Ansprüche: 6-22,24-30

   Nukleinsäure, kodierend für ein Polypeptid mit der Aktivität einer Fructose-1,6-biphosphat Aldolase aus pflanzenpathogenen Pilzen

3. Ansprüche: 23, 3 (teilweise)

   Verfahren zum Auffinden einer Verbindung, welche die Expression von Polypeptiden gemäss Anspruch 18 oder 19 verändert

**SEQUENCE LISTING**

<110> Bayer AG

<120> Use of fructose-1,6-bisphosphate aldolase for identifying new fungicidally active substances

<130> Le A 35 348

<160> 5

<170> PatentIn version 3.1

<210> 1
<211> 2002
<212> DNA
<213> Ustilago maydis

<220>
<221> CDS
<222> (97)..(207)
<223>

<220>
<221> CDS
<222> (430)..(1392)
<223>

```
<400>  1
atggtacgct gcaacaattc tggagcacag caaagctgac atgtaggcct tttccctttc      60

cttttctttg atcttttttg gtctccttgc ctcaga atg ggt gtt ctt gac att      114
                                        Met Gly Val Leu Asp Ile
                                        1               5

gtt ccc gcc ggt gtt gtc tcc gga aag gac gtt tac aag gtc ttc gac      162
Val Pro Ala Gly Val Val Ser Gly Lys Asp Val Tyr Lys Val Phe Asp
            10                  15                  20

tac gct cgt cag aac aag ttt gcc atc ccc gcc ttc aac gtt act          207
Tyr Ala Arg Gln Asn Lys Phe Ala Ile Pro Ala Phe Asn Val Thr
        25                  30                  35

gtaagtaccg cctttggctc aaggaaatgg tgtctcggga cgcggactgc cagcttctcg    267

cgagctgctt tctgtgataa aaaccttttc agattcaatg acaaaagtcg cataaattcg    327

ccctgttgga tgattgctga cgacctcatc gcattctctc gcatttcaac tacttcatgg    387

ataccgctgg cgaatctgcc gtttttgcga tttctgaaat ag tcg tcc tcg gtc       441
                                               Ser Ser Ser Val
                                                       40
```

```
gcc atc tcg gct ctc gag gct gct cgt gat gcc aag tcg ccc ctg atc       489
Ala Ile Ser Ala Leu Glu Ala Ala Arg Asp Ala Lys Ser Pro Leu Ile
            45                  50                  55

ctt cag gtc tca cag gga ggt gcc gcc aac ttt gcc ggc aag ggt ctc       537
Leu Gln Val Ser Gln Gly Gly Ala Ala Asn Phe Ala Gly Lys Gly Leu
        60                  65                  70

tct aac tcg aac cag gag gcc tcg atc atc ggt gcc aag gcc gcc gct       585
Ser Asn Ser Asn Gln Glu Ala Ser Ile Ile Gly Ala Lys Ala Ala Ala
    75                  80                  85

ctc ttc att cgc gcc gtt gct ccc tcg tac ggt gtc cct gtt atc atg       633
Leu Phe Ile Arg Ala Val Ala Pro Ser Tyr Gly Val Pro Val Ile Met
90                  95                  100                 105

cac tcg gac cac tgc gcc aag aag ctg ctt cct tgg ttt gat ggc atg       681
His Ser Asp His Cys Ala Lys Lys Leu Leu Pro Trp Phe Asp Gly Met
                110                 115                 120

ctc gcc gcc gac gag gag tac tac aag gag cac aac gag ccg ctc ttt       729
Leu Ala Ala Asp Glu Glu Tyr Tyr Lys Glu His Asn Glu Pro Leu Phe
            125                 130                 135

tcg tcg cac atg ctc gac tta tcc gag gag tcc aag gag gag aac atc       777
Ser Ser His Met Leu Asp Leu Ser Glu Glu Ser Lys Glu Glu Asn Ile
        140                 145                 150

gag acc tgc ctc aag tac ctc aag cgc atg gct cct ctc ggc atc tgg       825
Glu Thr Cys Leu Lys Tyr Leu Lys Arg Met Ala Pro Leu Gly Ile Trp
    155                 160                 165

ctc gag atg gag atc ggc att act ggt ggt gaa gag gac ggt gtt aac       873
Leu Glu Met Glu Ile Gly Ile Thr Gly Gly Glu Glu Asp Gly Val Asn
170                 175                 180                 185

aac gag ggt gtt gac aac gcc tcg ctc tac acc cag ccc gag gac atc       921
Asn Glu Gly Val Asp Asn Ala Ser Leu Tyr Thr Gln Pro Glu Asp Ile
                190                 195                 200

tgg gac atc tac cgc cag ttc tcc gag att acc ccc aac ttc tcc atc       969
Trp Asp Ile Tyr Arg Gln Phe Ser Glu Ile Thr Pro Asn Phe Ser Ile
            205                 210                 215

gcc gcc ggt ttc ggt aac gtt cac ggt gtc tac aag ccc gga aac gtc      1017
Ala Ala Gly Phe Gly Asn Val His Gly Val Tyr Lys Pro Gly Asn Val
        220                 225                 230

tcg ctg cag ccc gag ctg ctc ggc aag cac cag gag cac gtc cgc gct      1065
Ser Leu Gln Pro Glu Leu Leu Gly Lys His Gln Glu His Val Arg Ala
    235                 240                 245

cag atc aag agc gac aag gag aac ccg gtc ttc ttc gtc ttc cac ggc      1113
Gln Ile Lys Ser Asp Lys Glu Asn Pro Val Phe Phe Val Phe His Gly
250                 255                 260                 265

gga tcc ggt tcc gag aag cac gag atc agc acc gcc gtc agc cac ggt      1161
Gly Ser Gly Ser Glu Lys His Glu Ile Ser Thr Ala Val Ser His Gly
                270                 275                 280
```

```
gtg gtc aag atg aac gtc gac acc gac acg cag tgg gcc tac ctg ctc       1209
Val Val Lys Met Asn Val Asp Thr Asp Thr Gln Trp Ala Tyr Leu Leu
            285                 290                 295

ggt atc cgc gac tac atc ttg aac aag aag gac tac ctc atg agc cag       1257
Gly Ile Arg Asp Tyr Ile Leu Asn Lys Lys Asp Tyr Leu Met Ser Gln
        300                 305                 310

gtc ggc aac ccc gat ggc gcc gac aag cct aac aag aag ttc ttc gac       1305
Val Gly Asn Pro Asp Gly Ala Asp Lys Pro Asn Lys Lys Phe Phe Asp
    315                 320                 325

cct cgt gtt tgg gtt cgc gag ggt gag aag acc atg gct acg cgt tgc       1353
Pro Arg Val Trp Val Arg Glu Gly Glu Lys Thr Met Ala Thr Arg Cys
330                 335                 340                 345

aaa gag gcc ttt act gac ctt ggc tcc act ggc aag ctc taagctgttc        1402
Lys Glu Ala Phe Thr Asp Leu Gly Ser Thr Gly Lys Leu
                350                 355

acttgacaga agccattgac tatgttttcg aaggatggca cgtacacgta cacacacatg   1462

caaacacatg caaacacatg caaaatttga taatcttagc ccgatctgct ttgccaatac   1522

ggcgcgtgcc ggtgcattgc gtggagtaga cagatgtgag gttcaggaac gtggagctta   1582

gcaggcgtat tctgggcaga cgacgacgag tgggtaagct tgagatgcaa aggtcatgga   1642

tctggagacc agaaggtgca tctgcccgat gcgtttcctg ttaccagcct tgcgggcagc   1702

acggggtgca tacacgtaac agcggggacg gcggtgacca ggttctcgtc ccacaagctt   1762

ctgaggagcg tgccgtcggc agcgtacact tcggctcttc ggctcatgct gccgatggta   1822

aagtgaggtt cgagcaaagc gttttggttc catttggcgc ggaagagggt gagccatttc   1882

cctgtttggt tgttgtgtgg gatgcgcgtc gggttggcga gaacgtcgtc tgggcgcttc   1942

gagagggatg ggctaggtgc gtcctgttct tgcttgatgg cttcagcgac gtctttttc    2002


<210>  2
<211>  37
<212>  PRT
<213>  Ustilago maydis


<400>  2
Met Gly Val Leu Asp Ile Val Pro Ala Gly Val Val Ser Gly Lys Asp
1               5                   10                  15

Val Tyr Lys Val Phe Asp Tyr Ala Arg Gln Asn Lys Phe Ala Ile Pro
            20                  25                  30

Ala Phe Asn Val Thr
        35
```

<210> 3
<211> 321
<212> PRT
<213> Ustilago maydis

<400> 3
Ser Ser Ser Val Ala Ile Ser Ala Leu Glu Ala Ala Arg Asp Ala Lys
1 5 10 15

Ser Pro Leu Ile Leu Gln Val Ser Gln Gly Gly Ala Ala Asn Phe Ala
20 25 30

Gly Lys Gly Leu Ser Asn Ser Asn Gln Glu Ala Ser Ile Ile Gly Ala
35 40 45

Lys Ala Ala Ala Leu Phe Ile Arg Ala Val Ala Pro Ser Tyr Gly Val
50 55 60

Pro Val Ile Met His Ser Asp His Cys Ala Lys Lys Leu Leu Pro Trp
65 70 75 80

Phe Asp Gly Met Leu Ala Ala Asp Glu Glu Tyr Tyr Lys Glu His Asn
85 90 95

Glu Pro Leu Phe Ser Ser His Met Leu Asp Leu Ser Glu Glu Ser Lys
100 105 110

Glu Glu Asn Ile Glu Thr Cys Leu Lys Tyr Leu Lys Arg Met Ala Pro
115 120 125

Leu Gly Ile Trp Leu Glu Met Glu Ile Gly Ile Thr Gly Gly Glu Glu
130 135 140

Asp Gly Val Asn Asn Glu Gly Val Asp Asn Ala Ser Leu Tyr Thr Gln
145 150 155 160

Pro Glu Asp Ile Trp Asp Ile Tyr Arg Gln Phe Ser Glu Ile Thr Pro
165 170 175

Asn Phe Ser Ile Ala Ala Gly Phe Gly Asn Val His Gly Val Tyr Lys
180 185 190

Pro Gly Asn Val Ser Leu Gln Pro Glu Leu Leu Gly Lys His Gln Glu
195 200 205

His Val Arg Ala Gln Ile Lys Ser Asp Lys Glu Asn Pro Val Phe Phe
210 215 220

```
Val Phe His Gly Gly Ser Gly Ser Glu Lys His Glu Ile Ser Thr Ala
225                 230                 235                 240

Val Ser His Gly Val Val Lys Met Asn Val Asp Thr Asp Thr Gln Trp
                245                 250                 255

Ala Tyr Leu Leu Gly Ile Arg Asp Tyr Ile Leu Asn Lys Lys Asp Tyr
            260                 265                 270

Leu Met Ser Gln Val Gly Asn Pro Asp Gly Ala Asp Lys Pro Asn Lys
        275                 280                 285

Lys Phe Phe Asp Pro Arg Val Trp Val Arg Glu Gly Glu Lys Thr Met
    290                 295                 300

Ala Thr Arg Cys Lys Glu Ala Phe Thr Asp Leu Gly Ser Thr Gly Lys
305                 310                 315                 320

Leu


<210>  4
<211>  1077
<212>  DNA
<213>  Ustilago maydis


<220>
<221>  CDS
<222>  (1)..(1074)
<223>


<400>  4
atg ggt gtt ctt gac att gtt ccc gcc ggt gtt gtc tcc gga aag gac       48
Met Gly Val Leu Asp Ile Val Pro Ala Gly Val Val Ser Gly Lys Asp
1               5                   10                  15

gtt tac aag gtc ttc gac tac gct cgt cag aac aag ttt gcc atc ccc       96
Val Tyr Lys Val Phe Asp Tyr Ala Arg Gln Asn Lys Phe Ala Ile Pro
            20                  25                  30

gcc ttc aac gtt act tcg tcc tcg gtc gcc atc tcg gct ctc gag gct      144
Ala Phe Asn Val Thr Ser Ser Ser Val Ala Ile Ser Ala Leu Glu Ala
        35                  40                  45

gct cgt gat gcc aag tcg ccc ctg atc ctt cag gtc tca cag gga ggt      192
Ala Arg Asp Ala Lys Ser Pro Leu Ile Leu Gln Val Ser Gln Gly Gly
    50                  55                  60

gcc gcc aac ttt gcc ggc aag ggt ctc tct aac tcg aac cag gag gcc      240
Ala Ala Asn Phe Ala Gly Lys Gly Leu Ser Asn Ser Asn Gln Glu Ala
65                  70                  75                  80
```

```
tcg atc atc ggt gcc aag gcc gcc gct ctc ttc att cgc gcc gtt gct     288
Ser Ile Ile Gly Ala Lys Ala Ala Ala Leu Phe Ile Arg Ala Val Ala
                85                  90                  95

ccc tcg tac ggt gtc cct gtt atc atg cac tcg gac cac tgc gcc aag     336
Pro Ser Tyr Gly Val Pro Val Ile Met His Ser Asp His Cys Ala Lys
            100                 105                 110

aag ctg ctt cct tgg ttt gat ggc atg ctc gcc gcc gac gag gag tac     384
Lys Leu Leu Pro Trp Phe Asp Gly Met Leu Ala Ala Asp Glu Glu Tyr
        115                 120                 125

tac aag gag cac aac gag ccg ctc ttt tcg tcg cac atg ctc gac tta     432
Tyr Lys Glu His Asn Glu Pro Leu Phe Ser Ser His Met Leu Asp Leu
    130                 135                 140

tcc gag gag tcc aag gag gag aac atc gag acc tgc ctc aag tac ctc     480
Ser Glu Glu Ser Lys Glu Glu Asn Ile Glu Thr Cys Leu Lys Tyr Leu
145                 150                 155                 160

aag cgc atg gct cct ctc ggc atc tgg ctc gag atg gag atc ggc att     528
Lys Arg Met Ala Pro Leu Gly Ile Trp Leu Glu Met Glu Ile Gly Ile
                165                 170                 175

act ggt ggt gaa gag gac ggt gtt aac aac gag ggt gtt gac aac gcc     576
Thr Gly Gly Glu Glu Asp Gly Val Asn Asn Glu Gly Val Asp Asn Ala
            180                 185                 190

tcg ctc tac acc cag ccc gag gac atc tgg gac atc tac cgc cag ttc     624
Ser Leu Tyr Thr Gln Pro Glu Asp Ile Trp Asp Ile Tyr Arg Gln Phe
        195                 200                 205

tcc gag att acc ccc aac ttc tcc atc gcc gcc ggt ttc ggt aac gtt     672
Ser Glu Ile Thr Pro Asn Phe Ser Ile Ala Ala Gly Phe Gly Asn Val
    210                 215                 220

cac ggt gtc tac aag ccc gga aac gtc tcg ctg cag ccc gag ctg ctc     720
His Gly Val Tyr Lys Pro Gly Asn Val Ser Leu Gln Pro Glu Leu Leu
225                 230                 235                 240

ggc aag cac cag gag cac gtc cgc gct cag atc aag agc gac aag gag     768
Gly Lys His Gln Glu His Val Arg Ala Gln Ile Lys Ser Asp Lys Glu
                245                 250                 255

aac ccg gtc ttc ttc gtc ttc cac ggc gga tcc ggt tcc gag aag cac     816
Asn Pro Val Phe Phe Val Phe His Gly Gly Ser Gly Ser Glu Lys His
            260                 265                 270

gag atc agc acc gcc gtc agc cac ggt gtg gtc aag atg aac gtc gac     864
Glu Ile Ser Thr Ala Val Ser His Gly Val Val Lys Met Asn Val Asp
        275                 280                 285

acc gac acg cag tgg gcc tac ctg ctc ggt atc cgc gac tac atc ttg     912
Thr Asp Thr Gln Trp Ala Tyr Leu Leu Gly Ile Arg Asp Tyr Ile Leu
    290                 295                 300

aac aag aag gac tac ctc atg agc cag gtc ggc aac ccc gat ggc gcc     960
Asn Lys Lys Asp Tyr Leu Met Ser Gln Val Gly Asn Pro Asp Gly Ala
305                 310                 315                 320
```

```
gac aag cct aac aag aag ttc ttc gac cct cgt gtt tgg gtt cgc gag      1008
Asp Lys Pro Asn Lys Lys Phe Phe Asp Pro Arg Val Trp Val Arg Glu
                325                 330                 335

ggt gag aag acc atg gct acg cgt tgc aaa gag gcc ttt act gac ctt      1056
Gly Glu Lys Thr Met Ala Thr Arg Cys Lys Glu Ala Phe Thr Asp Leu
            340                 345                 350

ggc tcc act ggc aag ctc taa                                          1077
Gly Ser Thr Gly Lys Leu
        355


<210>  5
<211>  358
<212>  PRT
<213>  Ustilago maydis


<400>  5
Met Gly Val Leu Asp Ile Val Pro Ala Gly Val Val Ser Gly Lys Asp
1               5                   10                  15

Val Tyr Lys Val Phe Asp Tyr Ala Arg Gln Asn Lys Phe Ala Ile Pro
            20                  25                  30

Ala Phe Asn Val Thr Ser Ser Ser Val Ala Ile Ser Ala Leu Glu Ala
        35                  40                  45

Ala Arg Asp Ala Lys Ser Pro Leu Ile Leu Gln Val Ser Gln Gly Gly
    50                  55                  60

Ala Ala Asn Phe Ala Gly Lys Gly Leu Ser Asn Ser Asn Gln Glu Ala
65                  70                  75                  80

Ser Ile Ile Gly Ala Lys Ala Ala Ala Leu Phe Ile Arg Ala Val Ala
                85                  90                  95

Pro Ser Tyr Gly Val Pro Val Ile Met His Ser Asp His Cys Ala Lys
            100                 105                 110

Lys Leu Leu Pro Trp Phe Asp Gly Met Leu Ala Ala Asp Glu Glu Tyr
        115                 120                 125

Tyr Lys Glu His Asn Glu Pro Leu Phe Ser Ser His Met Leu Asp Leu
    130                 135                 140

Ser Glu Glu Ser Lys Glu Glu Asn Ile Glu Thr Cys Leu Lys Tyr Leu
145                 150                 155                 160
```

```
Lys Arg Met Ala Pro Leu Gly Ile Trp Leu Glu Met Glu Ile Gly Ile
                165                 170                 175

Thr Gly Gly Glu Glu Asp Gly Val Asn Asn Glu Gly Val Asp Asn Ala
            180                 185                 190

Ser Leu Tyr Thr Gln Pro Glu Asp Ile Trp Asp Ile Tyr Arg Gln Phe
        195                 200                 205

Ser Glu Ile Thr Pro Asn Phe Ser Ile Ala Ala Gly Phe Gly Asn Val
    210                 215                 220

His Gly Val Tyr Lys Pro Gly Asn Val Ser Leu Gln Pro Glu Leu Leu
225                 230                 235                 240

Gly Lys His Gln Glu His Val Arg Ala Gln Ile Lys Ser Asp Lys Glu
                245                 250                 255

Asn Pro Val Phe Phe Val Phe His Gly Gly Ser Gly Ser Glu Lys His
            260                 265                 270

Glu Ile Ser Thr Ala Val Ser His Gly Val Val Lys Met Asn Val Asp
        275                 280                 285

Thr Asp Thr Gln Trp Ala Tyr Leu Leu Gly Ile Arg Asp Tyr Ile Leu
    290                 295                 300

Asn Lys Lys Asp Tyr Leu Met Ser Gln Val Gly Asn Pro Asp Gly Ala
305                 310                 315                 320

Asp Lys Pro Asn Lys Lys Phe Phe Asp Pro Arg Val Trp Val Arg Glu
                325                 330                 335

Gly Glu Lys Thr Met Ala Thr Arg Cys Lys Glu Ala Phe Thr Asp Leu
            340                 345                 350

Gly Ser Thr Gly Lys Leu
        355
```